(19) Europäisches Patentamt
European Patent Office
Office européen des brevets

(11) EP 2 213 293 A2

(12) EUROPEAN PATENT APPLICATION

(43) Date of publication:
**04.08.2010 Bulletin 2010/31**

(21) Application number: **08022463.7**

(22) Date of filing: **07.12.2006**

(51) Int Cl.:
***A61K 31/785*** *(2006.01)* ***C08G 18/08*** *(2006.01)*
***C08G 18/28*** *(2006.01)* ***C08G 18/62*** *(2006.01)*

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK RS**

(30) Priority: **08.12.2005 US 748216 P**

(62) Document number(s) of the earlier application(s) in accordance with Art. 76 EPC:
**06851302.7 / 1 959 971**

(71) Applicant: **The Polymer Technology Group Incorporated**
**Berkeley, CA 94710 (US)**

(72) Inventors:
- **Ward, Robert S.**
  **Lafayette, California 94549 (US)**
- **McCrea, Keith R.**
  **Concord, California 94518 (US)**
- **Tian, Yuan**
  **Alameda, California 94501 (US)**
- **Parakka, James P.**
  **San Bruno, California 94066 (US)**
- **Wang, Shanger**
  **Fairfield, California 94534 (US)**

(74) Representative: **Duffy, Assumpta Dympna et al**
**FRKelly**
**27 Clyde Road**
**Ballsbridge**
**Dublin 4 (IE)**

Remarks:
This application was filed on 24-12-2008 as a divisional application to the application mentioned under INID code 62.

# (54) Self-assembling monomers and oligomers as surface-modifying endgroups for polymers

(57) Polymers having the formula $R(LE)_x$ wherein R is a polymeric core having a number average molecular weight of from 5000 to 7,000,000 daltons and having x endgroups, E is an endgroup covalently linked to polymeric core R by linkage L, L is a divalent oligomeric chain, having at least 5 identical repeat units, capable of self-assembly with L chains on adjacent molecules of the polymer, and the moieties $(LE)_x$ in the polymer may be the same as or different from one another. Design of monomers, oligomers, or other reactive structures otherwise analogous to known Self Assembled Monolayers but with at least one reactive chemical group capable of binding them to the terminus of a polymer, so that the thiol-free SAM analogue becomes the self-assembling surface modifying endgroup of that polymer. Use of the polymer to fabricate a configured article from the surface-modified polymer or a coating or topical treatment on an article made from another material.

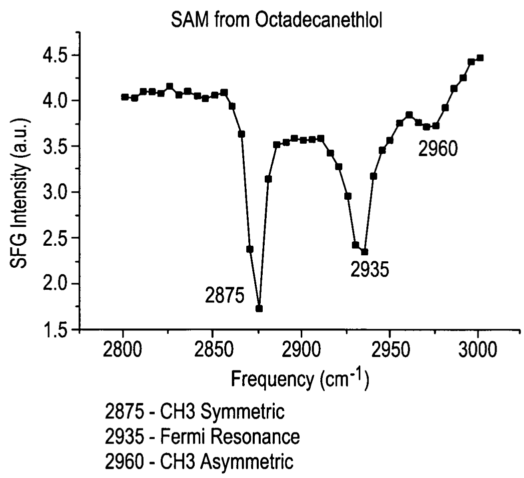


FIG.1A

EP 2 213 293 A2

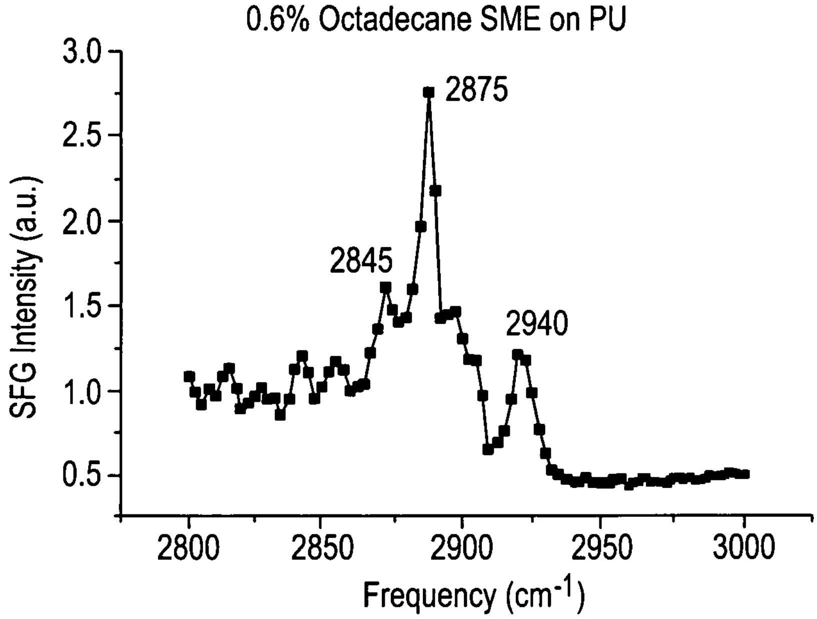


2845 - CH2 Symmetric (PU)
2875 - CH3 Symmetric (SME)
2940 - Fermi Resonance

FIG.1B

## Description

### FIELD OF THE INVENTION

This invention provides novel methods that enable the configuration of the nanostructure, supramolecular structure, and/or conformation of a molecular monolayer at the surface of a polymer body. This invention also provides novel articles of manufacture that employ the novel methods of the invention to enhance their suitability for use in medical and other applications. Finally, this invention provides novel polymers suitable for making the novel articles of the invention.

### BACKGROUND OF THE INVENTION

Work by the present inventors with polymers containing Surface Modifying Endgroups (SMEs) has compared them to surfaces created by adsorbing thiol monomers onto flat gold substrates to form so-called self-assembled monolayers (SAMs). SAM technology is discussed extensively in the literature, and SAMs have become very popular in research because of the relative ease with which well-defined surfaces can be created therewith. Although biomedical applications are of considerable interest in this regard, SAM methodology can be used to create surfaces for use in many other fields as well, including: sensors, electronics, microlithography, combinatorial chemistry, diagnostics, ship and aircraft coatings, etc. U.S. patent application US 2005/0282997 A1 (Serial No. 11/211,734), incorporated by reference herein, teaches additional applications involving polymer surface technology.

SAM monomers are generally applied to previously formed surfaces by adsorption from solution. It is known that thiols in solution bond to gold. This bond formation is an important factor driving the self assembly of the thiol monomers. In addition, at high packing density of the SAM monomer, it appears that hydrophobic interactions among alkane 'spacer chains' in alkane thiols also contribute to self assembly.

### SUMMARY OF THE INVENTION

We have found that when certain monomers that do not contain thiol groups are used as endgroups on linear polymers, they self assemble to form surfaces that behave like surfaces created by analogous thiol-containing SAM monomers on gold. The thiol-free, SAM-like endgroups, however, do not contain gold, nor do they require gold to be present to form self-assembled monolayers at the polymer surface.

Whereas thiol SAMs are attached to pre-formed gold substrates by fragile gold-thiol bonds, SMEs can be attached to polymers by stable covalent bonds during the initial synthesis of the polymer. That is, the use of SAM-like SMEs to obtain self assembly in the surface of a polymer does not require application of a coating after the original surface is created. Accordingly, SME-created surfaces in accordance with the present invention are much more robust and useful in practical applications than are conventional SAM-created surfaces. For instance, an SAM surface created from a thiol monomer adsorbed onto gold may last only a few hours or days after its formation. In contrast, an SME-created polymer surface can be extremely robust and resistant to degradation. Furthermore, unlike conventional SAM coatings, SME-containing polymers can be strong structural materials useful in the fabrication of components and other configured items.

SMEs are effective when present at very low bulk concentration in the polymer to be modified. For example, useful polymer surface modification may be obtained when SMEs comprise < 1 weight percent of the total polymer mass. Although certain SMEs may require higher concentrations, for instance when *bulk* polymer modification is also a goal, the low level of bulk concentration of SMEs necessary to effect useful polymer modification generally does not affect the original polymer's processability and physical-mechanical properties. In some cases, in fact, SMEs may actually enhance processing, for instance by providing internal lubrication to molten or dissolved polymer chains.

For these reasons, SME-containing polymers may be processed by a wide range of commercially-viable manufacturing methods. Molding, extrusion, and all other thermoplastic methods of 'conversion' can be used to form SME-containing polymers into useful articles. Solvent-based processing, water-borne systems, and 100%-solids (crosslinkable) liquid or gum processing can also be used to fabricate useful articles from SME-containing polymers. SAMs, on the other hand, are difficult or impossible to apply in many manufacturing processes.

Another advantage of SME-polymers relative to topical treatments such as chemisorbed thiols and silane monomers is that in SME-containing polymers, the surface-modifying moiety can be present on virtually every molecule of the bulk polymer. Furthermore, even at low bulk concentrations and the surface-to-volume ratios typical of most formed articles, there is a considerable reserve or reservoir of SMEs *within* the bulk of the polymeric article. This reservoir of SMEs is available to replace SMEs that might be lost during use, for instance by abrasion. In contrast, conventional SAMS, by design, consist of a single monomolecular layer on a typically rigid substrate. If the conventional SAM is damaged, the substrate is exposed and self-healing is unlikely without re-application of the SAM monomer.

The present invention provides a means for employing the benefits of SAM technology in practical industrial, medical, and consumer applications. This includes the development of a robust and easily processed polymeric material which spontaneously 'present' a surface similar or identical to that provided by SAMs used in research. We have disclosed methods of using homopathic SMEs in US 5,589,563 and amphipathic SMEs in our published PCT patent application WO 2004/044012 A1 and in application US 2005/0282997 A1. The present application includes method claims as set forth below. Although a primary focus of the present invention is in biomedical applications, the present invention is by no means limited to biomedical applications.

One benefit of the present invention involves the use of SAMs in laboratory or exploratory studies, and/or the use of SAM research results from the literature, to optimize the chemistry and structure of a polymer surface for a particular application. In accordance with the present invention, the benefits of SAMs in preparing well-defined surfaces (previously mostly confined to the laboratory) are available at this stage. This benefit of the present invention may involve the use of one or more simple SAMs with spacers and functional endgroups groups, with a thiol, silane, or other group at the end of the spacer chain - opposite to the end thereof at which the functional endgroup or head group is located - that binds it to a substrate. This 'SAM research' may optionally involve a SAM to which another (biologically) active, biomimetic, or functional group is attached in an optional reaction step that follows self assembly onto the substrate as described below.

Another embodiment of this invention is the design of monomers, oligomers, or other reactive structures otherwise analogous to the SAM but with at least one reactive chemical group capable of binding it to the terminus of the polymer to be modified, so that the thiol-free SAM analogue becomes the (self-assembling surface modifying) endgroup of that polymer. In accordance with this invention, the actual identity of the reactive group that couples the SAM analogue to the polymer will be determined by the chemistry of the polymer to be modified, e.g., an active hydrogen-containing group, an olefinic group, a silane group, an acryloxy or methacryloxy group, etc., depending on the reactions, catalysts, and methods used to bind the SME to the polymer, which in turn depends upon the monomers used to synthesize the main chains of the polymer to be modified.

Another embodiment of this invention is the synthesis and optional purification of the SME-containing polymer with the specified endgroups.

Another embodiment of this invention is the use of the SME polymer to fabricate a configured article from the surface-modified polymer, or a coating or topical treatment on an article made from another material. In accordance with this invention, any of the available methods of polymer fabrication can be used, including thermoplastic, solvent-based, water-based dispersions, evaporative depositions, sputtering, dipping, painting, spraying, 100%-solids single component or multi-component processing, machining, thermo-forming, cold forming, etc.

Another embodiment of this invention is allowing the configured article to spontaneously develop the surface of interest by the diffusion/migration of the endgroups to the surface of the configured article and self assembly of those endgroups in the surface. In accordance with this invention, environmental conditions - for maximizing the rate of self assembly and/or the quality of the self-assembled monolayer - can be determined with the optional use of sensitive, surface-specific analytical methods like Sum Frequency Generation Vibrational Spectroscopy (SFG), contact angle goniometry, Atomic Force Microscopy, etc., or through the use of functional testing of the surface after preparation using the candidate environmental condition(s): for instance, time, temperature, and the nature of the fluid or solid in contact with the polymer surface. Functional testing of candidate surface/pretreatment combinations may be done in the actual application in which the surface will be used, or by use of an *in vitro* test that predicts performance of the surface in the actual application.

Another embodiment of this invention is the optional binding of functional, biomimetic, and/or (biologically) active moieties to the surface optimized as described above, or to the non-optimized surface of the configured article produced as described above.

Another embodiment of this invention is the use of the configured article. Many examples of applications are given in the 'Amphipathic SME' patent application. Such applications can be used with the novel polymers of the present invention as well. Accordingly, the entire disclosures of applications US 2005/0282997 A1 and WO 2004/044012 A1 are expressly incorporated by reference herein.

This invention provides polymers having the formula

$$R(LE)_x$$

wherein R is a polymeric core having a number average molecular weight of from 5000 to 7,000,000 daltons, more usually up to 5,000,000 daltons, and having x endgroups, x being an integer ≥1, E is an endgroup covalently linked to polymeric core R by linkage L, L is a divalent oligomeric chain, having at least 5 identical repeat units, capable of self-assembly with L chains on adjacent molecules of the polymer, and, when x > 1, the moieties $(LE)_x$ in the polymer may be the same as or different from one another, although in many cases, all of the moieties $(LE)_x$ in the polymer are the same as one another.

In these polymers having the formula

$$R(LE)_x$$

L, for instance, may be a divalent alkane, polyol, polyamine, polysiloxane, or fluorocarbon of from 8 to 24 units in length.

In these polymers having the formula

$$R(LE)_x$$

E may be an endgroup that is positively charged, negatively charged, or that contains both positively charged and negatively charged moieties. Also, E may be an endgroup that is hydrophilic, hydrophobic, or that contains both hydrophilic and hydrophobic moieties. Also, E may be a biologically active endgroup, such as heparin. In this embodiment, E may be a heparin binding endgroup such as PDAMA or the like that is linked to the polymer backbone via a self assembling polyalkylene spacer of different chain lengths, typically between 8 and 24 units. In another embodiment, E may be an antimicrobial moiety, such as a quaternary ammonium molecules as disclosed in US 6,492,445 B2 (expressly incorporated herein by reference) or an oligermeric compounds such as a poly quat derivatized from an ethylenically unsaturated diamine and an ethylenically unsaturated dihalo compound. The antimicrobial moiety may be an organic biocidal compound that prevents the formation of a biological microorganism, and has fungicidal, algicidal, or bactericidal activity and low toxicity to humans and animals, e.g., a quaternary ammonium salt that bears additional reactive functional group capable of attaching to the polymer main chain, such as compounds having the following formula:

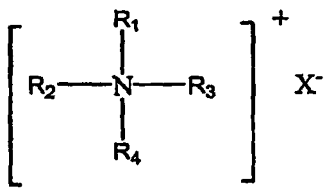

wherein $R_1$, $R_2$, and $R_3$ are radicals of straight or branched or cyclic alkyl groups having one to eighteen carbon atoms or aryl groups and $R_4$ is an amino-, hydroxyl-, isocyanato-, vinyl-, carboxyl-, or other reactive group-terminated alkyl chain capable of covalently bonding to the base polymer, wherein, due to the permanent nature of the immobilized organic biocide, the polymer thus prepared does not release low molecular weight biocide to the environment and has long lasting antimicrobial activity. Alternatively, E may be an amino group, an isocyanate group, a hydroxyl group, a carboxyl group, a carboxaldehyde group, or an alkoxycarbonyl group. Thus, E may be a protected amino group linked to the polymer backbone via a self assembling polyalkylene spacer of different chain lengths, typically between 8 and 24 units. In some specific embodiments, E may be selected from the group consisting of hydroxyl, carboxyl, amino, mercapto, azido, vinyl, bromo, acrylate, methacrylate, $-O(CH_2CH_2O)_3H$, $-(CH_2CH_2O)_4H$, $-O(CH_2CH_2O)_6H$,$-O(CH_2CH_2O)_6CH_2COOH$, $-O(CH_2CH_2O)_3CH_3$, $-(CH_2CH_2O)_4 CH_3$, $-O(CH_2CH_2O)_6CH_3$, trifluoroacetamido, trifluoroacetoxy, 2',2',2'-trifluorethoxy, and methyl.

In these polymers having the formula

$$R(LE)_x$$

R typically (although not invariably) has a number average molecular weight of from 100,000 to 1,000,000 daltons. R may be, for example, a linear base polymer when x is 2, E is a surface active endgroup, and L is a polymethylene chain of the formula $-(CH_2)_{n-}$ wherein n is an integer of from 8 to 24. In some embodiments, the linear base polymer may be a polyurethane and the endgroup may be a monofunctional aliphatic polyol, an aliphatic or aromatic amine, or mixtures thereof. In many embodiments of the present invention, R will be biodegradable and/or bioresorbable.

In these polymers having the formula

$$R(LE)_x$$

in some embodiments, at least some of the moieties $(LE)_x$ in the polymer may be different from other of the moieties $(LE)_x$ in the polymer. In this embodiment of the present invention, the spacer chains may be of different lengths, the endgroups may have different molecular weights and/or identities, or both the spacer chains and the endgroups may be different from one another. One practical application of the varied surface that this embodiment imparts to the polymer would be, for instance, improved 'rejection' of both low and high molecular weight proteins when immersed in sea water

or body fluids. Using two or more different spacer chain chemistries which self assemble but do not assemble with spacer chains of different chemistry would produce a "patchy" monolayer at the polymer surface (useful e.g. in certain applications for discouraging protein adsorption). An example of this is a polyurethane or polyurea polymer in which about half of the moieties $(LE)_x$ in the polymer have E groups derived from a polyethylene oxide having a molecular weight of about 2000 and the reactive monomer that forms the endgroup has the formula $HO(CH_2)_{17}(CH_2CH_2O)_{45}CH_3$, and about half of the moieties $(LE)_x$ in the polymer have E groups that are derived from a polyethylene oxide having a molecular weight of about 5000 and the reactive monomer that forms the endgroup has the formula

$$HO(CH_2)_{17}(CH_2CH_2O)_{114}CH_3.$$

Another class of embodiments of the present invention is medical device or prosthesis or packaging assembly comprising a polymer body, wherein the polymer body comprises a plurality of polymer molecules located internally within said body, at least some of which internal polymer molecules have endgroups that comprise a surface of the body, wherein the surface endgroups include at least one self-assembling monolayer moiety, wherein the polymer comprising the self-assembling molecular moieties in the polymer body is a first polymer making up the entirety of a major portion of the body and having a weight average molecular weight in the range 5000-5,000,000 daltons (preferably 50,000-5,000,000 daltons.), or is a second polymer, having a weight average molecular weight in the range 1000-500,000 daltons, which comprises an additive to the first polymer making up the entirety or a major portion of the body. The device or prosthesis in these embodiments configured as an implantable medical device or prosthesis or as a non-implantable disposable or extracorporeal medical device or prosthesis or as an *in vitro* or *in vivo* diagnostic device, wherein said device or prostheses has a tissue, fluid, and/or blood-contacting surface. In these devices or prostheses, the polymer body may be a dense or microporous membrane component in an implantable medical device or prosthesis or in a non-implantable disposable or extracorporeal medical device or prosthesis or as an *in vitro* or *in vivo* diagnostic device, and wherein, when said polymer body comprises a membrane component in a diagnostic device, said component contains immuno-reactants.

The device or prosthesis of this invention can comprise a blood gas sensor, a compositional sensor, a substrate for combinatorial chemistry, a customizable active biochip, a semiconductor-based device for identifying and determining the function of genes, genetic mutations, and proteins, a drug discovery device (wherein the drug is complexed to surface-modifying endgroups and is released through diffusion or wherein the drug is associated with, complexed to, or covalently bound to surface-modifying endgroups that degrade and release the drug over time), an immunochemical detection device, a glucose sensor, a pH sensor, a blood pressure sensor, a vascular catheter, a cardiac assist device, a prosthetic heart valve, an artificial heart, a vascular stent, a prosthetic spinal disc, a prosthetic spinal nucleus, a spine fixation device, a prosthetic joint, a cartilage repair device, a prosthetic tendon, a prosthetic ligament, a drug delivery device from which drug molecules are released over time, a drug delivery coating in which drugs are fixed permanently to polymer endgroups, a catheter balloon, a glove, a wound dressing, a blood collection device, a blood storage container, a blood processing device, a plasma filter, a plasma filtration catheter, a device for bone or tissue fixation, a urinary stent, a urinary catheter, a contact lens, an intraocular lens, an ophthalmic drug delivery device, a male condom, a female condom, devices and collection equipment for treating human infertility, a pacemaker lead, an implantable defibrillator lead, a neural stimulation lead, a scaffold for cell growth or tissue engineering, a prosthetic or cosmetic breast implant, a prosthetic or cosmetic pectoral implant, a prosthetic or cosmetic gluteus implant, a penile implant, an incontinence device, a laparoscope, a vessel or organ occlusion device, a bone plug, a hybrid artificial organ containing transplanted tissue, an *in vitro* or *in vivo* cell culture device, a blood filter, blood tubing, roller pump tubing, a cardiotomy reservoir, an oxygenator membrane, a dialysis membrane, an artificial lung, an artificial liver, or a column packing adsorbent or chelation agent for purifying or separating blood, plasma, or other fluids.

The device or prosthesis of the present invention, when configured as an implantable medical device or prosthesis or as a non-implantable disposable or extracorporeal medical device or prosthesis or as an *in vitro* or *in vivo* diagnostic device, may optionally have antimicrobial activity afforded by self-assembling antimicrobial agents covalently bonded to the polymer chain as an endgroup.

A packaging assembly of the present invention may include a polymer body, wherein the polymer body comprises a plurality of polymer molecules located internally within said body, at least some of which internal polymer molecules have endgroups that comprise a surface of the body, wherein the surface endgroups include at least one self-assembling monolayer moiety, wherein the polymer comprising the self-assembling monolayer moieties in the polymer body is a first polymer making up the entirety of a major portion of the body and having a weight average molecular weight in the range 5000-5,000,000 daltons, or is a second polymer, having a weight average molecular weight in the range 1000-500,000 daltons, which comprises an additive to the first polymer making up the entirety or a major portion of the body, or wherein said packaging assembly comprises a plastic bottle and eyedropper assembly containing a sterile solution, wherein said self-assembling monolayer moieties bind an antimicrobial agent and wherein said bound antimicrobial agents maintain the sterility of said solution.

This invention also provides a method of immobilizing biologically-active entities, including proteins, peptides, and polysaccharides, at a surface of a polymer body, which polymer body surface comprises a surface of an interface, which method comprises the sequential steps of contacting the polymer body surface with a medium that delivers self-assembling monolayer moieties containing chemically-reactive groups, capable of binding biologically-active entities to the surface, to the polymer body surface by interaction of chemical groups, chains, or oligomers, said self-assembling monolayer moieties being covalently or ionically bonded to a polymer in the body and comprising one or more chemical groups, chains, or oligomers that spontaneously assemble in the outermost monolayer of the surface of the polymer body or one or more chemical groups, chains, or oligomers that spontaneously assemble within that portion of the polymer body that is at least one monolayer away form the outermost monolayer of the polymer body surface, and binding said biologically-active entities to said reactive groups, wherein the polymer comprising the self-assembling monolayer moieties in the polymer body is a first polymer making up the entirety of a major portion of the body and having a weight average molecular weight in the range 5000-5,000,000 daltons (preferably at least 50,000 daltons), or is a second polymer, having a weight average molecular weight in the range 1000-500,000 daltons, which comprises an additive to the first polymer making up the entirety or a major portion of the body, or wherein said self-assembling monolayer moieties containing binding groups comprise methoxy ether-terminated polyethyleneoxide oligomers having one or more amino, hydroxyl, carboxaldehyde, or carboxyl groups along the polyethyleneoxide chain. In this embodiment, the polymer comprising the self-assembling monolayer moieties in the polymer body may be a first polymer making up the entirety of a major portion of the body and having a weight average molecular weight in the range 5000-5,000,000 daltons, or may be a second polymer, having a weight average molecular weight in the range 1000-500,000 daltons, which comprises an additive to the first polymer making up the entirety or a major portion of the body.

BRIEF DESCRIPTION OF THE DRAWINGS

Figure 1 shows SFG spectra for (a) octadecanethiol SAM and (b) octadecane SMEs on BIONATE ® 55D polycarbonate-urethane (PCU).

Figure 2 shows the surface concentration increase of octadecane SMEs as a function of time while the formed article is allowed to equilibrate at room temperature.

Figure 3 shows SFG results illustrating the effect of solvents on SAM-like SME surface assembly.

Figure 4 shows the effect of annealing SAM-like SME samples of Example 3.

DETAILED DESCRIPTION OF THE INVENTION

As noted above, this invention provides a class of polymers having the general formula

$$R(LE)_x$$

in which R is a polymeric core having x endgroups, E is an endgroup covalently linked to polymeric core R by linkage L, and L is a divalent oligomeric chain capable of self-assembly with L chains on adjacent molecules of the polymer. Before providing a detailed description of broader aspects of the present invention, we present the following non-limiting illustrative specific examples of polymers provided by the present invention.

The polymeric composition of matter illustrated below, wherein R is a polydimethylsiloxane base polymer having a MW of 500,000 daltons, L is $-Si(CH_3)_2-(CH_2)_{12}-O-C(CH_3)_2-$, E is 2000 dalton MW polyvinylpyrrolidone, and x is 2.

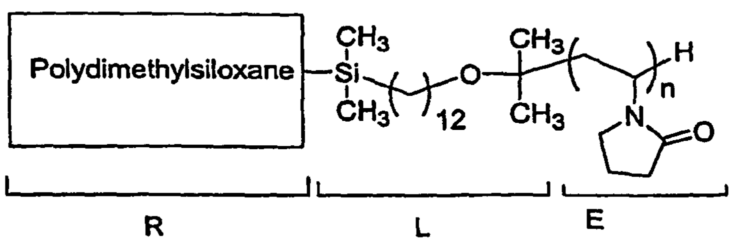

The polymeric composition of matter illustrated below, wherein R is a polyetherurethane base polymer having a MW of 250,000 daltons, L is $NH-C(=O)-O-(CH_2)_{10}-O-C(CH_3)_2-$ , E is 1000 dalton MW polyvinylpyrrolidone, and x is 2.

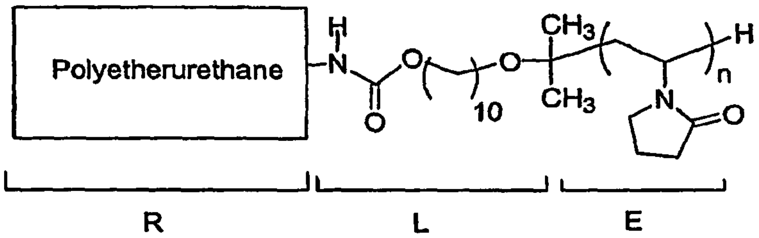

The polymeric composition of matter illustrated below, wherein R is a polycarbonate urethane polymer having a MW of 500,000 daltons, L is -NH-C(=O)-O-$(CH2)_8$-, E is PDAMA, and x is 2.

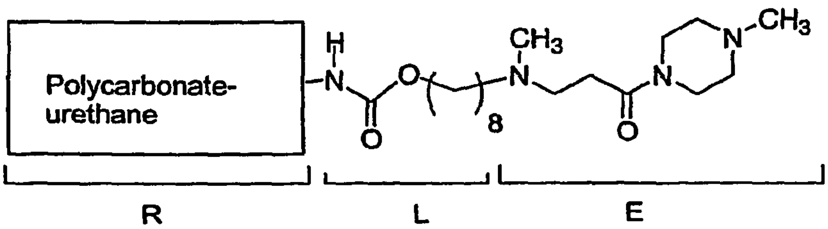

The polymeric composition of matter illustrated below, wherein R is a polyurethane-polyurea copolymer having a MW of 250,000 daltons, L is -NH-C(=O)-NH-$(CH_2)_{16}$-NH-$CH_2$-, E is heparin, and x is 2.

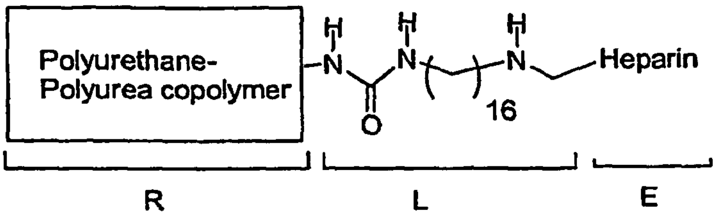

The polymeric composition of matter illustrated below, wherein R is a polyetheretherketone base polymer having a MW of 300,000 daltons, L is -O-$[Si(CH_3)_2O]_{16}$-$CH_2$-$CH_2$-O-$C(CH_3)_2$-, E is 2000 dalton MW polyvinylpyrrolidone, and x is 2.

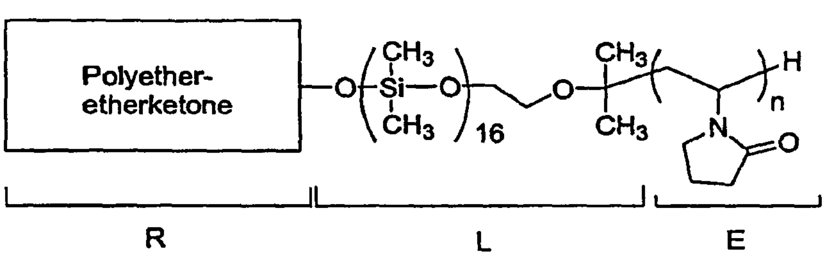

The polymeric composition of matter illustrated below, wherein R is a polymethylmethacrylate base polymer having a MW of 500,000 daltons, L is -C(=O)O-$(CH_2)_{11}$-O-, E is PhC, and x is 1.

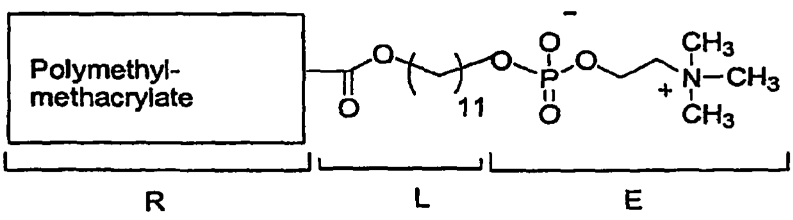

The polymeric composition of matter illustrated below, wherein R is a polyurethane-polyurea copolymer having a MW of 300,000 daltons, L is -NH-C(=O)-NH-$(CH_2)_{12}$-NH-C(=O)-, E is a RGD peptide, and x is 2.

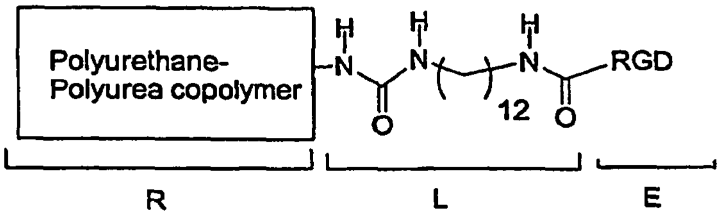

The polymeric composition of matter illustrated below, wherein R is a polyetherurethane base polymer having a MW of 250,000 daltons, L is NH-C(=O)-[O-$(CH_2)_2$-O]$_4$-O-C$(CH_3)_2$-, E is 1000 dalton MW polyvinylpyrrolidone, and x is 2.

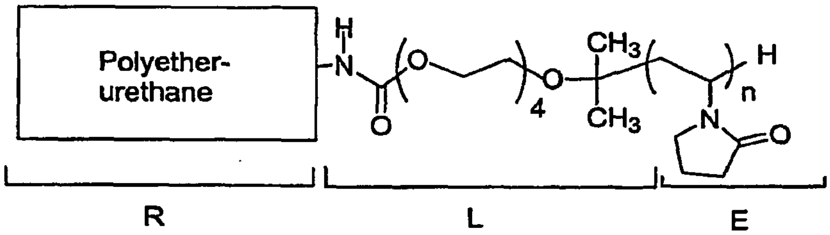

The polymeric composition of matter illustrated below, wherein R is a polydimethylsiloxane base polymer having a MW of 400,000 daltons, L is -O-$CH_2$-$CH_2$-OOC$(CH_3)_2$-PVP with n=10 repeat units, E is a methacrylate reactive group, and x is 2.

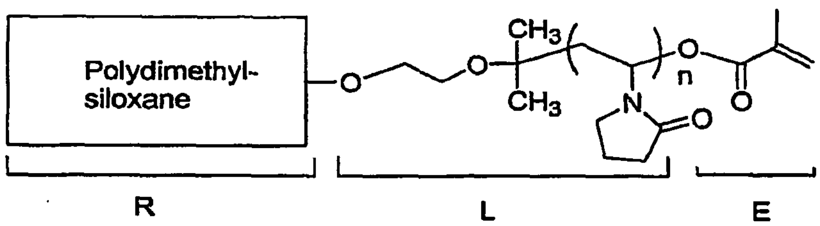

The polymeric composition of matter illustrated below, wherein R is a polyetherurethane base polymer having a MW of 300,000 daltons, L is NH-C(=O)-O-$(CH_2)_3$ [Si$(CH_3)_2$O]$_{10}$-$(CH_2)_3$-O-C(=O)-NH-$(CH_2)_6$-NH-C(=O)-, E is isethionic acid ($HOCH_2CH_2SO_3H$), and x is 2.

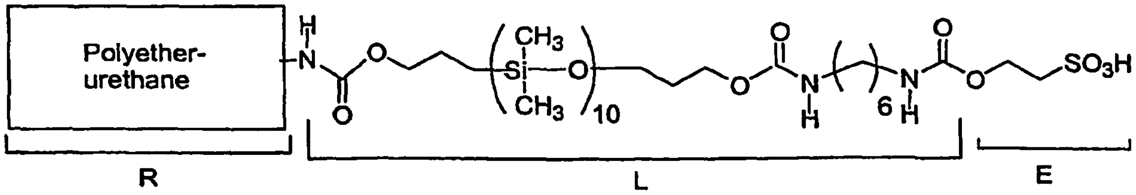

The polymeric composition of matter illustrated below, wherein R is a polyetherurethane base polymer having a MW of 300,000 daltons, L is -NH-C(=O)-O-$(CH_2)_3$ $[Si(CH_3)_2O]_{10}$-$(CH_2)$3-O-C(=0)-NH-$(CH_2)_6$-NH-C(=O)-, E is isethionic acid sodium salt ($HOCH_2CH_2SO_3Na$), and x is 2.

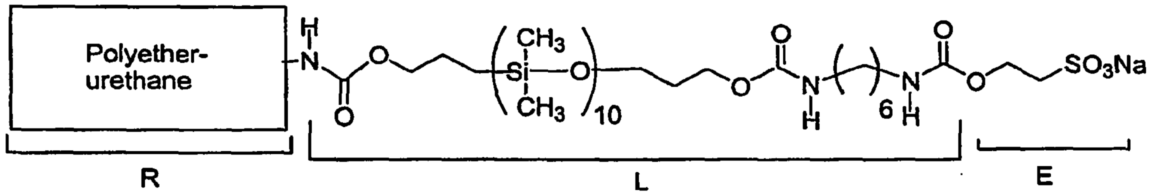

The polymeric composition of matter illustrated below, wherein R is a polyurethane polydimethylsiloxane copolymer having a MW of 200,000 daltons, L is -NH-C(=O)-NH-$(CH_2)_8$-, E is $NH_2$, and x is 2.

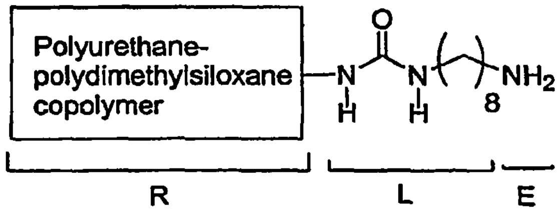

The polymeric composition of matter illustrated below, wherein R is a polystyrene base polymer having a MW of 400,000 daltons, L is -$[Si(CH_3)_2O]_{10}$-$Si(CH_3)_2$-$CH_2$-$CH_2$-$CH_2$-O-$CH_2$-, E is oxirane (epoxide) reactive group, and x is 1.

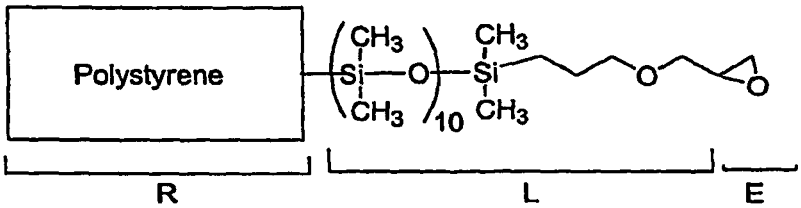

The polymeric composition of matter illustrated below, wherein R is a n-butylpolydimethylsiloxane having a MW of 1,000 daltons, L is -PVP-$CH_2CH_2$- with n =10 repeat units, E is a reactive methacrylate, and x is 1.

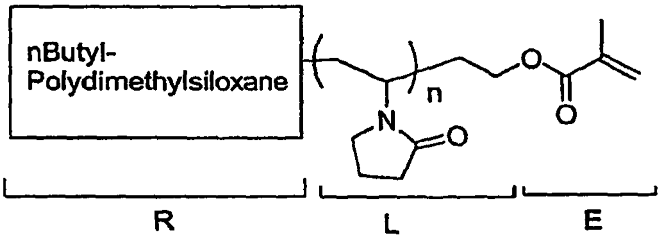

The polymeric composition of matter illustrated below, wherein R is a polyetherurethane base polymer having a MW of 200,000 daltons, L is a polybutadiene crosslinkable spacer, $-NH-C(=O)-O-(CH_2-CH=CH-CH_2)_{12}-O-$, E is $CH_3$ group and x is 2.

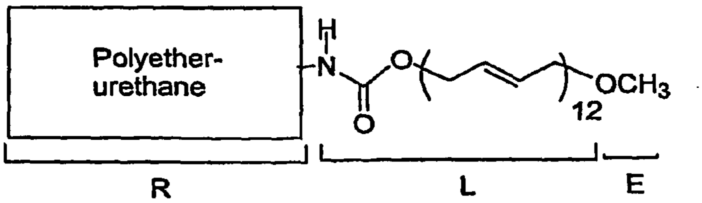

The polymeric composition of matter illustrated below, wherein R is a polyurethane-polyurea copolymer having a MW of 250,000 daltons, L is $NH-C(=O)-NH-(CH_2)_{12}-NH-C(=O)-$, E is L-DOPA (3,4-dihydroxy-L-phenylalanine), and x is 2.

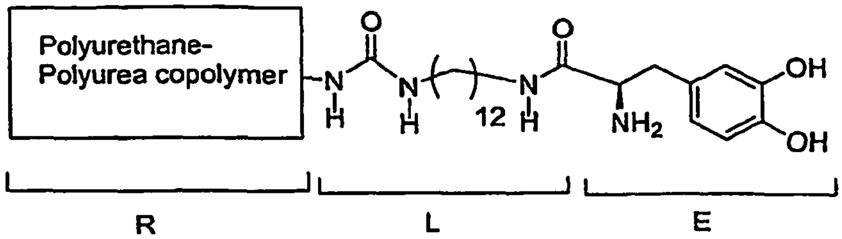

The polymeric composition of matter illustrated below, wherein R is a polyetherurethane base polymer having a MW of 200,000 daltons, L is $NH-C(=O)-O-(CH_2)_{12}-(OCH_2CH_2)_4-O-C(=O)-$, E is L-DOPA (3,4-dihydroxy-L-phenylalanine), and x is 2.

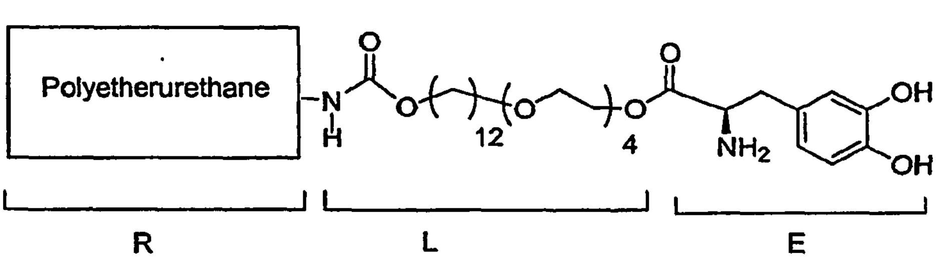

The polymeric composition of matter illustrated below, wherein R is a "branched" polyetherurethane base polymer having a MW of 200,000 daltons, L is $-NH-C(=O)-NH-(CH_2)_8-$, E is an amine ($NH_2$) group, and x is 4. The branched polymer is obtained by making use of pentaerythritol $C(CH_2OH)_4$ for the synthesis with structure illustrated

below.

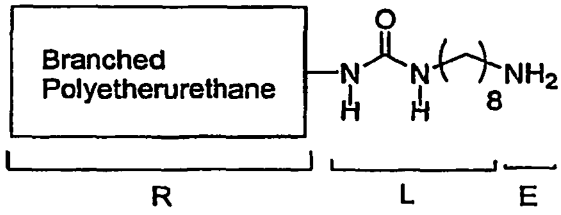

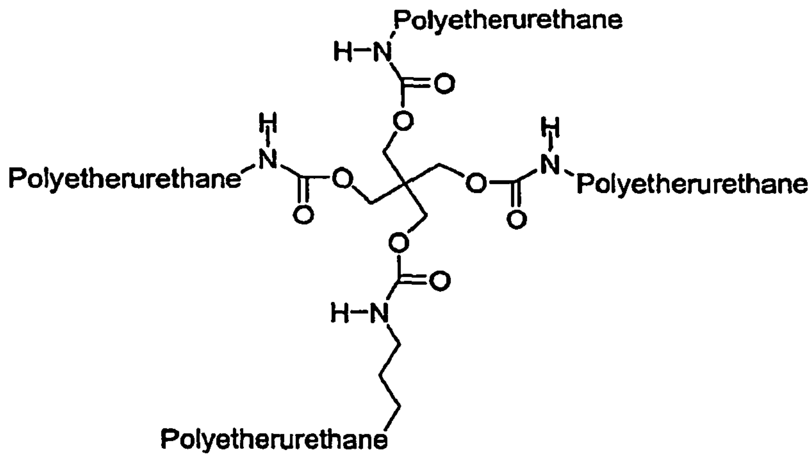

Branched polyetherurethane.

US 5,589,563 (Robert S. Ward and Kathleen A. White) describes the use of surface modifying endgroups (SMEs) to tailor polymer surface properties. The '563 patent is entitled "SURFACE-MODIFYING ENDGROUPS FOR BIOMEDICAL POLYMERS". The entire contents of US 5,589,563 are hereby expressly incorporated by reference. As documented in the '563 patent, a variety of simple hydrophobic and hydrophilic endgroups has been demonstrated to enable the achievement of useful changes in surface properties of polymers. Such surface properties include biostability, protein adsorption, abrasion resistance, bacterial adhesion and proliferation, fibroblast adhesion, and coefficient of friction. SME polymers have also been used in low bulk concentration as surface modifying additives (SMAs) to SME-free base polymers. Polymers of the types disclosed in US 5,589,563 may be used as base polymers for carrying the covalently bonded Self-Assembling Monolayer endgroups of the present invention. US 2005/0282977 A1 (Robert S. Ward, Keith R. McCrea, Yuan Tian, and James P. Parakka) also discloses polymers that may be used as base polymers in the present invention. The entire contents of US 2005/0282997 A1 are hereby expressly incorporated by reference.

A "self-assembling moiety"-containing polymer molecule endgroup is defined as an endgroup that spontaneously rearranges its positioning in a polymer body to position the moiety on the surface of the body, which positioning effects a reduction in interfacial energy. The endgroup structure may comprise one or more chemical groups, chains, or oligomers that spontaneously assemble in the outermost monolayer of the surface of the polymer body, or may comprise one or more chemical groups, chains, or oligomers that spontaneously assemble within the bulk of the polymer body. The polymer bulk is defined as the region within the polymer body that is at least one monolayer away from the outermost monolayer of the polymer body surface.

This invention provides a method of configuring the nanostructure, supramolecular structure, and/or conformation of a molecular monolayer at a surface of a polymer body at an interface. The method involves contacting the polymer body surface with a separate medium to form an interface under conditions that facilitate the delivery of endgroup molecular moieties to the polymer body surface and maximize the resulting concentration of head groups in the outermost surface. This delivery is, in part, due to the interaction of chemical groups, chains, or oligomers in the endgroup moieties. The endgroup molecular moieties are covalently or ionically bonded to a polymer in the body and include one or more

chemical groups, chains, or oligomers that spontaneously assemble in the outermost monolayer of the surface of the polymer body or one or more chemical groups, chains, or oligomers that spontaneously assemble within that portion of the polymer body that is at least one monolayer away from the outermost monolayer of the polymer body surface. In accordance with the present invention, the endgroups are bonded to the polymers through a divalent oligomeric chain, having at least 5 repeat units, that is capable of self-assembly with corresponding chains on adjacent molecules of the polymeric composition. Suitable structures for the spacer chains can be found in the SAM and silane literature. In general, self-assembing spacer chains suitable for polymer endgroups of the presnet invention will be those that self assemble when present in self-assembling thiol or silane SAMs. Accordingly persons skilled in the art of conventional SAM monomers, e.g., on gold or silicon substrates, can readily determine suitable spacer chains for use in making the self-assembling monomers which can be employed in the present invention.

In this method, the surface-modifying endgroup moieties may be delivered to the polymer body surface by their spontaneous diffusion *to* the surface region of the polymer body or by their rearrangement or repacking *in* the surface layer of the polymer body.

The polymer comprising the surface-modifying endgroup moieties in the polymer body makes up the entirety, or a major portion, of the body and has a weight average molecular weight in the range 5000-5,000,000 daltons, preferably in the range 50,000-1,000,000 daltons. Optionally, delivery of surface-modifying endgroups to the polymer body surface can be accomplished by adding a Surface-Modifying Additive (SMA) to the polymer just described, with the additive comprising a second polymer that is covalently or ionically bonded to the surface-modifying endgroup moieties of the present invention.

When delivery of the surface-modifying endgroup moiety to the polymer surface is accomplished by adding an SMA to the polymer to be modified, the useful molecular weight range of the polymer used as an SMA may be lower: 1000-5,000,000 daltons and preferably in the range 5000 to 200,000 daltons. This is because the SMA is typically used in low bulk concentrations, e.g. less than 15 weight-%, and preferably about 1 to 5 weight-%, so that the physical-mechanical properties of the base polymer/SMA blend will be largely determined by the base polymer being modified. However, very low SMA molecular weight may cause the SMA to be fugitive from the polymer being modified, e.g. by leaching or even volatilizing from the surface of the base polymer in use, particularly when there is exposure to fluids, vacuum, and/or high temperatures in use. Candidate SMA polymers with molecular weight less than 5000 are generally unsuitable and must be tested for their permanence in the base polymer before use in applications.

Alternatively, delivery of surface-modifying endgroup moieties to the polymer body surface or other substrate to be modified may be accomplished by coating, plasma treatment, painting, or otherwise topically treating the surface of a pre-formed body with a material comprising a second polymer covalently or ionically bonded to the surface-modifying endgroup moieties of the present invention.

Another method of this invention is the method of immobilizing enzymes, proteins, peptides, polysaccharides, or other biologically active or biomimetic moieties at an interfacial surface of a polymer body. This method comprises the sequential steps of (a) contacting the polymer body with a medium that facilitates delivery of endgroup molecular moieties to the surface which molecular moieties are capable of self assembling and are bonded to chemically-reactive groups capable of binding biologically-active entities to the surface of the polymer body, and (b) binding the enzymes, proteins, peptides, polysaccharides, or other biologically active or biomimetic moieties to the reactive groups in a suitable medium such as aqueous solution. The endgroup molecular moieties in the present invention are covalently or ionically bonded to a polymer in the body and comprise one or more chemical groups, chains, or oligomers that spontaneously assemble in the outermost monolayer of the surface of the polymer body.

## *SUM FREQUENCY GENERATION ANALYSIS*

Surface-Modifying Endgroups of the present invention are designed to migrate to an article's surface and to self assemble in that surface. The analysis required to investigate the chemical composition and orientation of a surface monolayer provided in this way, as well as surface monolayers on conventional SAMs, will ideally probe only that monolayer in order to obtain an accurate representation of the surface. Various spectroscopic techniques - including reflection infrared spectroscopy, attenuated total reflection infrared spectroscopy, and Raman spectroscopy - have been used to characterize polymer surfaces. These methods, however, lack surface specificity and the resulting spectra are often obscured by the response from the bulk. Surface-sensitive techniques such as contact angle measurement, neutron reflection, and X-ray photoelectron spectroscopy often do not provide structural information, and/or do not allow for *in situ* measurement. More recently, a surface-specific analytical technique with monolayer sensitivity has successfully been applied it to various kinds of surfaces and interfaces. Through IR and visible sum-frequency generation spectroscopy (SFG), a powerful and versatile *in situ* surface probe has been created that not only permits identification of surface molecular species, but also provides information about orientation of functional groups at the surface. SFG has the common advantages of laser techniques. That is, it is nondestructive, highly sensitive, and has good spatial, temporal, and spectral resolution.

During an SFG experiment, two laser beams are overlapped both in time and space on a polymer surface. The first laser is a fixed visible green beam with a wavelength of 532 nm ($\omega_{vis}$). The second laser is a tunable infrared beam ($\omega_{IR}$), e.g., in the wavelength range between 2 and 10 $\mu$m (1000 - 4000 $cm^{-1}$). The visible and IR beams mix on the surface to drive an oscillating dipole which then emits a coherent beam of photons at the sum of the visible and IR frequencies ($\omega_{SFG} = \omega_{vis} + \omega_{IR}$). A photo multiplier tube easily detects this generated beam to record a vibrational spectrum. Under the electric dipole approximation, the intensity of the sum frequency signal is proportional to the square of the second-order nonlinear surface susceptibility
($I \propto |X^{(2)}|^2$). The susceptibility is described by the equation

$$\chi^{(2)} = A_{NR} + \sum_{R} \frac{A_R}{(\omega_{IR} - \omega_0 - i\gamma)}$$

where $A_{NR}$ is the non-resonant contribution, $\gamma$ is the line width, $\omega_o$ is the resonant vibrational frequency, and $\omega_{IR}$ is the IR frequency. The resonant strength, $A_R$, is proportional to the concentration and orientation of molecules on the surface and the infrared and Raman transition moments. As observed in this equation, when $\omega_{IR}$ is equal to $\omega_o$, $X^{(2)}$ is maximized and so a surface vibrational spectrum can be obtained by scanning $\omega_{IR}$ through a frequency range of interest. Since $A_R$ is proportional to the IR and Raman transition moments, the selection rules for both IR and Raman spectroscopy must be obeyed. Hence, a media must be both IR-active and Raman-active. From group theory, it can be shown that only media that lack inversion symmetry will satisfy this requirement. Usually, bulk materials are centrosymmetric and therefore do not generate SFG. Isotropic gasses and liquids also do not generate SFG. Only at surfaces or interfaces where the centrosymmetry of the bulk material is broken can SFG occur, therefore, SFG is extremely surface specific.

SFG is surface specific for many polymers because the bulk is amorphous; there is no net orientation of the polymer chains. Because of this random orientation, $X^{(2)}$ vanishes, and SFG is not allowed. A polymer surface, however, can have a net orientation of backbone atoms or functional groups at its surface, which leads to polar ordering. $X^{(2)}$ is then non-zero for a polymer surface, and is therefore SFG allowed. The orientation of molecules at the surface can also be determined by SFG. As described earlier, $X^{(2)}$ is proportional to the orientation of surface molecules. $X^{(2)}$ is a third rank tensor and the net orientation of surface molecules can be deduced by probing the surface with different polarizations of light. By changing the polarization of the input and output beams, different components of the tensor are accessed.

Because SFG is surface specific, the technique can be used to probe any interface as long as the media the laser beams must pass through do not interfere with the light. Examples of the interfaces accessible by SFG include but are not limited to the polymer/gas interface and the polymer/liquid interface

The SFG apparatus is a complex laser system based on a high-power picosecond Nd:YAG laser and an optical parametric generator/amplifier (OPG/OPA). The fundamental output (1064 nm) of the Nd:YAG laser is frequency doubled to produce the 532 nm visible beam and is used to drive an OPO/OPA. The tunable (e.g., 1000 to 4000 $cm^{-1}$) IR beam is generated from a series of non-linear crystals through OPG/OPA and difference frequency mixing. The sum-frequency (SF) spectra are obtained by overlapping the visible and IR beams on the polymer surface at incident angles of 55° and 60°, respectively. The SF signal from the polymer surface is filtered by a monochromator, collected by a photomultiplier tube (PMT), and processed using gated integrator. Surface vibrational spectra are obtained by measuring the SF signal as a function of the input IR frequency.

EXAMPLES

Relative to backbone chains, polymer endgroups are more mobile allowing them to diffuse from the bulk, and assemble at the polymer interface relative to their bulk concentration. This produces major changes in surface composition that occurs spontaneously if the presence of the endgroups in the surface reduces system interfacial energy. Simple hydrophobic endgroups diffuse to an air interface, while purely hydrophilic endgroups enrich a polymer surface exposed to aqueous body fluids. These and more complex surface-modifying endgroups (SMEs) can be specifically tailored to affect the biologic response of polymers used in medical devices. For instance, in air, methoxy-terminated polyethylene oxide SMEs on a polyether-urethane polymers present a surface that is rich in hydrophobic methyl groups, but that surface is devoid of methyl groups in water. This is due to an endgroup conformation in which hydrated PEO 'arches' project from the surface, and terminal methyl groups are buried below the outermost surface layer accessable by Sum Frequency Generation (SFG). Other placements of hydrophobic groups and optional reactive groups on hydrophilic endgroups can produce more complex surface nanostructures useful in applications, including the delivery or permanent binding of biologically-active molecules.

EXAMPLE 1

Self-Assembling Monoloayer (SAM) of this Example prepared from octadecanethiol by absorption from ethanol solution onto a gold substrate. The 'SAM-containing polymer' with an aromatic polycarbonate-urethane (PCU) backbone is synthesized by continuous step growth polymerization on a twin screw extruder using a mono-functional SME analogue of the SAM monomer (octadecanol) as a chain stopper. That is, a reactive hydroxyl group 'replaces' the thiol group on octadecanethiol. During bulk polymer synthesis the SME is coupled to the ends of the polymer backbone by urethane linkages formed by reaction between hydroxyl groups on the octadecanol and isocyanate groups on the PCU polymer being modified. The monofunctionality of the octadecanol assures that it chain stops the polymer, forming an endgroup. A film of the fully reacted SME polymer is cast from solution on a continuous web coater. Both surfaces are characterized by SFG in air as described below.

The SME-PCU-SME polymer formed as described above is extremely tough. Tensile Strength is, for example, 62 Mpa. Ultimate Elongation is, for example, 400%. The SFG spectra for (a) octadecanethiol SAM and (b) octadecane SMEs on BIONATE ® 55D polycarbonate-urethane (PCU) are shown in **Figure 1**. The methyl symmetric and Fermi resonance peaks of octadecane are observed at 2875 and 2935 $cm^{-1}$, respectively. Although the bulk octadecane SME concentration in the PCU is only 0.6 wt%, the methyl peaks dominate the BIONATE SFG spectra, with only a small peak contributed by the methylenes present in the polycarbonate PCU backbone. In both plots the ordinate is SFG Intensity [a.u.], the abscissa is Frequency [$cm^{-1}$]. Note: Destructive interference between the non-resonant gold signal and resonant SAM vibrational signal creates negative peaks associated with SAM vibrational modes.

Initial SAM development on gold is often characterized by rapid formation of gold-thiol bonds and planar conformation of the alkane chains, followed by slower filling in of the final monolayer, attainment of the characteristic angle of the alkanes relative to the surface, and close packing of (e.g., methyl) head groups. In SME polymers the diffusion of endgroups from the bulk 'replaces' the SAM adsorption step, but it appears that the remaining steps toward surface equilibrium are similar. That is, upon arriving at the air interface from the bulk, the SAM-like SME may initially assume a planar conformation to maximize both the coverage by hydrophobic methylene groups, and the resulting interfacial energy reduction. As more SMEs arrive the alkanes begin to pack more closely in the surface and subsequently allow a tighter packing of very hydrophobic methyl groups, for an additional decrease in air/polymer interfacial energy. Polarized SFG measurements indicate that the equilibrium structure of the outermost, air-facing surface is composed of close-packed methyl head groups.

The concentration of the SAM-like SMEs at the surface depends on diffusion kinetics which is dependent on temperature. If a formed article is kept at room temperature, it may take several days for the surface diffusion of SMEs to be complete. **Figure 2** shows the surface concentration increase of octadecane SMEs as a function of time while the formed article is allowed to equilibrate at room temperature. At time 0, only a small peak attributed to the terminal methyl group is observed at 2875 $cm^{-1}$. As the sample is allowed evolve over time, the 2875 $cm^{-1}$ peak increases indicating an increase of octadecane at the surface.

Alkane thiol SAMs are assembled in various solvents to enhance assembly. Solvents also affect the assembly of SAM-like SMES. Ethanol is a polar solvent often used in SAM assembly. Octadecane SME containing articles were soaked for 24 hours at RT in each in ethanol. **Figure 3** shows SFG results illustrating the effect solvents have on SAM-like SME surface assembly. The 2875/2855 ratio gives the concentration of SME relative to BIONATE functional groups at the surface. The surface concentration of SME, relative to BIONATE groups, actually decreases if the film is exposed to ethanol. This shows that polar solvents can suppress assembly of non-polar SMEs (octadecane) just as polar solvents can enhance assembly of hydrophilic SMEs.

A hydrophobic solvent (hexane) was also used to treat an octadecane SME containing article. Because octadecane is hydrophobic, hexane will enhance the assembly of the SMEs at the surface as indicated by the 2875/2850 ratio increase. In addition, the ratio of the 2875 to 2960 peak gives us information about the orientation of the methyl groups. As the ratio increases, the methyl group becomes more perpendicular to the surface. This ratio is considerably larger for the hexane soaked sample as compared to the as received or ethanol soaked samples. Soaking hydrophobic SAM-like SME containing articles in polar solvents increases the rate of diffusion and packing of the SMEs at the surface. Non-polar solvents suppress assembly of hydrophilic SMEs.

Thermal annealing SAM-like SME containing articles also enhances assembly of the SME at the surface. **Figure 4** shows the effect of annealing the samples of Example 3, below. Annealing the untreated, ethanol treated, and hexane treated articles show enhancement in the assembly of the octadecane SME at the surface.

EXAMPLE 2

Synthesis of a SAM-containing polymer with an aromatic polycarbonate-urethane (pCU) backbone by step growth polymerization using mono-functional heparin binding compounds of the type (PDAMA) depicted below. The resulting polymer is populated with heparin binding sites on the surface as a result of self assembly of the polyalkylene

chain.
This Example generates PCU that bind to heparin via non-covalent interactions

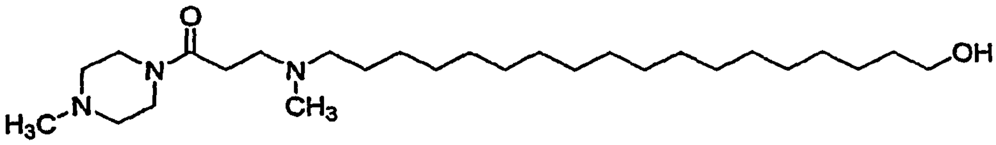

PDAMA-$C_{18}$-SME

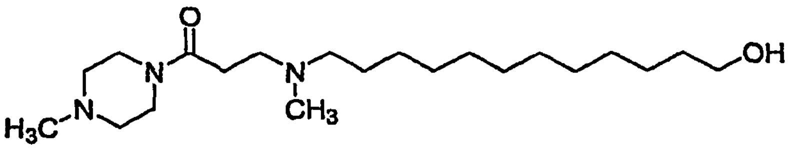

PDAMA-$C_{12}$-SME

EXAMPLE 3

Synthesis of a SAM-containing polymer with an aromatic polycarbonate-urethane (PCU) backbone by step growth polymerization and subsequent reaction with a compound bearing a Butyloxycarbonyl (BOC) protected amino group as shown below. De-protection under acidic conditions using organic acids (for e.g. trifluoracetic acid - $CH_2Cl_2$ mixture) or mineral acids (for e.g. dilute HCl) affords amino terminated PCU. Reaction of the said amino functionalized polymer with heparin aldehyde to form a Schiff's base and subsequent reduction generates a covalently bonded heparinized polymer with end-point attachment of the heparin.

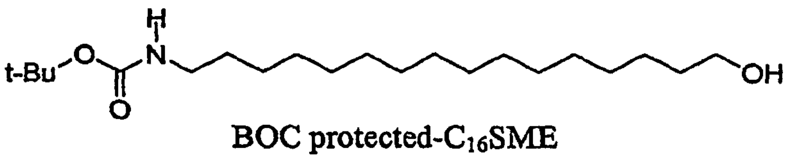

BOC protected-$C_{16}$SME

EXAMPLE 4

The synthesis of a 'SAM-containing polymer with an aromatic polycarbonate-urethane (PCU) backbone by step growth polymerization using mono-functional heparin binding compounds of the zwitterionic phosphoryl choline (PhC) type depicted below. The resulting polymer is populated with heparin binding sites on the surface as a result of self assembly of the polyalkylene chain. This example generates PCU that bind to heparin via ionic interactions. In addition, the quarternary amine group is a suitable endgroup that provides antimicrobial properties.

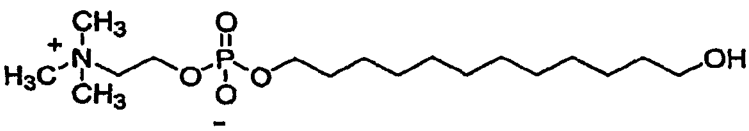

PhC-C12-SME

EXAMPLE 5

A thermoplastic polyurethane bearing antimicrobial functionality is described in the following formula, wherein PCU is polycarbonate urethane bulk chain, $R_1$, $R_2$, and $R_3$ are radicals of straight, branched, or cyclic alkyl groups having one to eighteen carbon atoms or aryl groups that are substituted or unsubstituted. $R_4$ is an amino, hydroxyl, isocynate, vinyl, carboxyl, or other reactive group terminated alkyl chain that react with polyurethane chemistry.

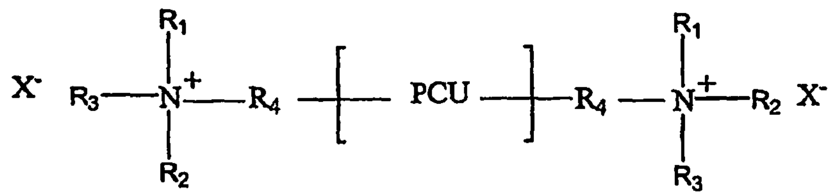

Illustrative of such suitable quaternary ammonium germicides for use in the invention is one prepared from N, N-trimethylamine and 2-chloroethyloxyethyloxyethanol to form a quaternary salt. This quaternary is used as a surface modifying endgroup (SME) in preparing thermoplastic polyurethanes (B) in bulk or in solution. Self assembly of this SME occurs at the surface through the intramolecular interaction of the glyme groups.

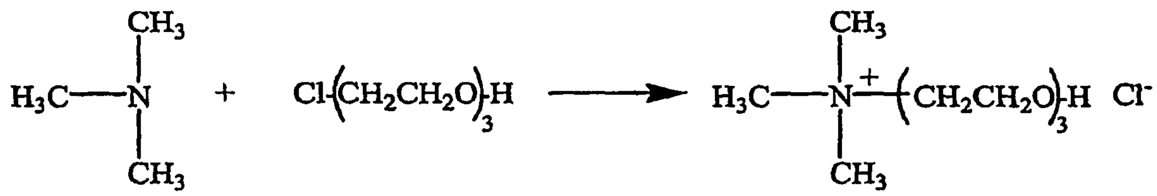

(A)

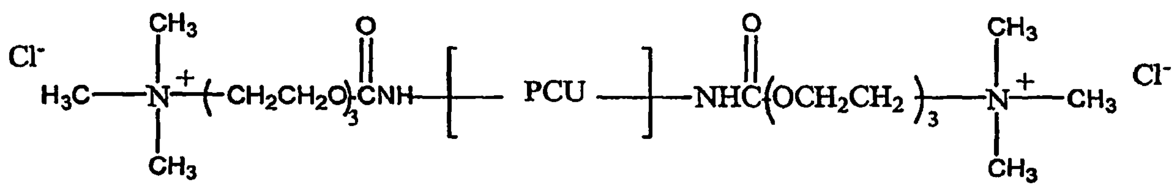

(B)

EXAMPLE 6

Thermoplastic polyurethanes bearing lubricious surface properties are described below. Hydroxyl terminated polyvinyl pyrrolidone (C) is prepared by the radical polymerization of vinyl pyrrolidone in the presence of a hydroxyl containing radical transfer agent. This prepared hydroxyl terminated PVP is used as surface modifying endgroup (SME) in preparing thermoplastic polyurethanes (D) in bulk or in solution. Self assembly at the surface occurs through the intramolecular forces between the the C12 alkane chain.

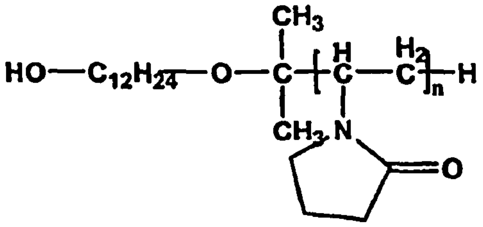

(C)

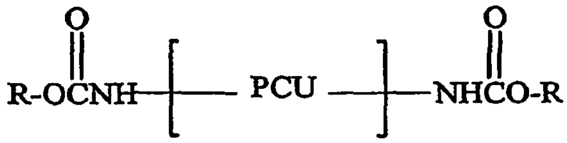

(D)

where R is

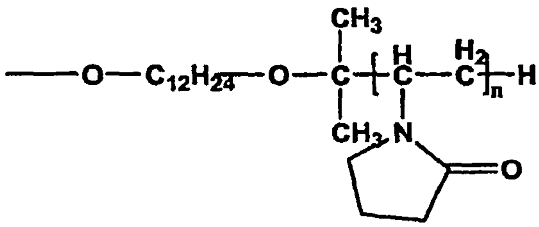

*APPLICATIONS OF THE NOVEL METHODS*

Unconfigured SAM-containing polymers of this invention may be converted to formed articles by conventional thermoplastic methods used to process polymers, including methods such as extrusion, injection molding, compression molding, calendering, and thermoforming under pressure or vacuum and stereo lithography. Multilayer processing such as co-extrusion or over-molding can be used on top of the base polymers to be economically viable and afford the surface properties from the SAM-containing polymer. SAM polymers may also be processed by solution-based techniques, such as air brush or airless spraying, ink jet printing, stereo lithography, elecrostatic spraying, brushing, dipping, casting, and coating. Water-based SAM polymer emulsions can be fabricated by methods similar to those used for solvent-based methods. In both cases, the evaporation of a volatile liquid (e.g., organic solvent or water) leaves behind a film of the SAM polymer. The present invention also contemplates the use of liquid or solid polymers with self assembling endgroups, optionally including or capable of binding biologicially active or biomimetic species, in computer-controlled stereolithography - also know as three dimensional printing. This method is of particular use in the fabrication of dense or porous structures for use in applications, or as prototypes, for tissue engineering scaffolds, prostheses, medical devices, artificial organs, and other medical, consumer, and industrial end uses.

Optionally, the polymer melt or liquid system may include reinforcing particulate fillers or pore formers that may be solid, liquid, or gaseous. Solid and liquid pore formers may be removed after component fabrication by well-known methods including water, solvent, or super-critical fluid extraction, gaseous diffusion, evaporation etc., to create porous structures in which the surface-modified pores may be isolated, interconnected, or reticulated, depending on the initial loading and size of the incorporated pore formers. Such porous structures are useful as tissue engineering substrates, filters, prostheses, membranes, weight-reduced structures, and many other well-known uses of porous media. The above, and other, fabrication considerations which are applicable to the present invention are discussed in US 5,589,563, the contents of which are hereby expressly incorporated by reference.

Often, surface-modifying endgroup moieties have little or no negative effect on processability. In fact, certain SAM-containing endgroups actually enhance processability of certain polymers that incorporate them by favorably impacting wetting and spreading by the base polymer on incorporated fillers, and on mandrels or polymeric, metallic, or

nonmetallic substrates to be coated. SAM-containing polymers may also provide improved mold release properties, internal lubricity among adjacent polymer chains, increased smoothness of extrudates, and lower viscosity of polymers during thermoplastic, solution, and water-based processing. Out-gassing and surface finish during solvent casting, coalescence of water-based emulsions, adhesion to substrates, and so on may also be improved in SAM-containing polymers, as compared to their unmodified analogues.

Polymers used to make useful articles in accordance with this invention will generally have tensile strengths of from about 100 to about 10,000 psi and elongations at break of from about 50 to about 1500%. In some particularly preferred embodiments, porous or non-porous films of the present invention are provided in the form of flexible sheets or in the form of hollow membranes or fibers made by melt blowing, spinning, electrostatic spraying, or dipping, for example. Typically, such flexible sheets are prepared as long rollable sheets of about 10 to 15 inches in width and 1 to hundreds of feet in length. The thicknesses of these sheets may range from about 5 to about 100 microns. Thicknesses of from about 19 to 25 microns are particularly useful when the article to be manufactured is to be used without support or reinforcement.

When membranes are fabricated from the polymers of this invention by knife-over-roll casting onto release paper, web, or a liner, for instance, a 24-foot-long 15-inch-wide continuous web coater equipped with forced-air ovens may be utilized. The coater may be modified for clean operation by fitting the air inlet ducts with High Efficiency Particulate Air filters. A nitrogen-purged coater box may be used to hold and dispense filtered polymer solutions or reactive prepolymer liquids. All but trace amounts of casting solvent (e.g., dimethylformamide) may be removed by the coater's hot air ovens fitted with HEPA filters. After membrane casting or another solvent-based fabrication method, the membrane and/or substrate may be further dried and/or extracted to reduce residual solvent content to less than about 100 ppm, for example. No significant loss of surface modifying moieties occurs during these post-fabrication purifications of SAM-containing polymers, because these moieties are covalently or ionically bonded to virtually every SAM-containing polymer molecule.

Polymer membranes of this invention may have any shape resulting from a process utilizing a liquid which is subsequently converted to a solid during or after fabrication, e.g., solutions, dispersion, 100% solids prepolymer liquids, polymer melts, etc. Converted shapes may also be further modified using methods such as die cutting, heat sealing, solvent or adhesive bonding, or any of a variety of other conventional fabrication methods.

In the case of thermoplastic surface-modifying endgroup moiety-containing polymers of this invention, thermoplastic fabrication methods may also be employed. Membrane polymers made by bulk or solvent-free polymerization method may be cast into, e.g., a Teflon-lined pan during the polymerization reaction. As the reaction proceeds and the polymerizing liquid becomes a rubbery solid, the pan may be post-cured in an oven, e.g. at 100-120°C for about an hour. Upon cooling, the solid mass may be chopped into granules and dried in a dehumidifying hopper dryer for, e.g., about 16 hours. The dry granules may then be compression molded, e.g., at about 175°C, to form a flat membrane which, when cool, will have a thickness of about 50 mm. Extrusion, injection molding, calendering, and other conversion methods that are well-known in the art may also be employed to form membranes, films, and coatings of the polymers of the present invention configured into solid fibers, tubing , medical devices, and prostheses. As those skilled in the art will appreciate, these conversion methods may also be used for manufacturing components for non-medical product applications.

This invention thus provides medical devices or prostheses which are constituted of polymer bodies, wherein the polymer bodies comprise a plurality of polymer molecules located internally within said body, at least some of which internal polymer molecules have endgroups that comprise a surface of the body. The polymer bodies can include dense, microporous, or macroporous membrane components in implantable medical devices or prostheses or in non-implantable disposable or extracorporeal medical devices or diagnostic products. For example, in one embodiment, the polymer body may comprises a membrane component or coating containing immuno-reactants in a diagnostic device. The present invention is particularly adapted to provide such articles configured as implantable medical devices or prostheses or as non-implantable disposable or extracorporeal medical devices or prostheses or as in *in vitro* or *in* vivo diagnostic devices, wherein the device or prostheses has a tissue, fluid, and/or blood-contacting surface.

Where the article of the present invention is a delivery device, a device for delivering drugs, including growth factors, cells, microbes, islets, osteogenic materials, neovascular-inducing moieties, the active agent may be complexed to the SAM endgroups and released through diffusion, or it may be complexed or bonded to SAM endgroups which are chosen to slowly degrade and release the drug over time. In accordance with this invention, the surface endgroups of the polymers include surface-modifying endgroup moieties, provided that at least some of said covalently bonded surface-modifying endgroup moieties are other than alkylene ether-terminated poly(alkylene oxides). These latter medical devices or prostheses are excluded from the present invention to the extent that they are disclosed in US 5,589,563.

Those skilled in the art will thus appreciate that the present invention provides improved blood gas sensors, compositional sensors, substrates for combinatorial chemistry, customizable active biochips - that is, semiconductor-based devices for use in identifying and determining the function of genes, genetic mutations, and proteins, in applications including DNA synthesis/diagnostics, drug discovery, and immunochemical detection, glucose sensors, pH sensors,

blood pressure sensors, vascular catheters, cardiac assist devices, prosthetic heart valves, artificial hearts, vascular stents and stent coatings, e.g., for use in the coronary arteries, the aorta, the vena cava, and the peripheral vascular circulation, prosthetic spinal discs, prosthetic spinal nuclei, spine fixation devices, prosthetic joints, cartilage repair devices, prosthetic tendons, prosthetic ligaments, drug delivery devices from which the molecules, drugs, cells, or tissue are released over time, delivery devices in which the molecules, drugs, cells, or tissue are fixed permanently to polymer endgroups, catheter balloons, gloves, wound dressings, blood collection devices, blood processing devices, plasma filters, plasma filtration catheters and membranes, devices for bone or tissue fixation or regrowth, urinary stents, urinary catheters, contact lenses, intraocular lenses, ophthalmic drug delivery devices, male and female condoms, devices and collection equipment for treating human infertility, insulation tubing and other components ofpacemaker leads and other electro-stimulation leads and components such as implantable defibrillator leads, neural stimulation leads, scaffolds for cell growth/regrowth or tissue engineering, prosthetic or cosmetic breast or pectoral or gluteal or penile implants with or without leak detection capability, incontinence devices, devices for treating acid reflux disease, devices for treating obesity, laparoscopes, vessel or organ occlusion devices, neurovascular stents and occlusion devices and related placement components, bone plugs, hybrid artificial organs containing transplanted tissue, *in vitro* or *in vivo* cell culture devices, blood filters, blood tubing, roller pump tubing, cardiotomy reservoirs, oxygenator membranes, dialysis membranes, artificial lungs, artificial livers, or column packing adsorbents or chelation agents for purifying or separating blood, blood cells, plasma, or other fluids. All such articles can be made by conventional means, with the benefits of this invention being provided by the surface-modifying endgroups that characterize the polymers described herein.

A variation of the above is plastic packaging for storing and/or dispensing sterile products. One example would be plastic bottles with optional eyedropper assemblies, which generally contain antimicrobial additives in addition to eye medication. In accordance with this invention, a polymer containing SAM-like endgroups that bind an antimicrobial or biocide such as benzalkonium chloride or Polyquad are incorporated into the packaging plastic, thus avoiding or reducing the need for such antimicrobial agents to be present in solution form within the packaging. Such packaging is useful for drugs, protein-based products, eye drops, contact lens solutions, contact lenses, and other ophthalmic devices for improving vision, protecting the eye, delivering drugs, treating dry eye, or for cosmetic/aesthetic uses.

Those skilled in the art are also well aware of how to use such embodiments of the present invention. See for instance: Ebert, Stokes, McVenes, Ward, and Anderson, Biostable Polyurethane Silicone Copolymers for Pacemaker Lead Insulation, The 28th Annual Meeting of the Society for Biomaterials, April 24-27, 2002, Tampa, Florida; Ebert, Stokes, McVenes, Ward, and Anderson, Polyurethane Lead Insulation Improvements using Surface Modifying Endgroups, The 28th Annual Meeting of the Society for Biomaterials, April 24-27, 2002, Tampa, Florida; Litwak, Ward, Robinson, Yilgor, and Spatz, Development of a Small Diameter, Compliant, Vascular Prosthesis, Proceedings of the UCLA Symposium on Molecular and Cell Biology, Workshop on Tissue Engineering, February, 1988, Lake Tahoe, California; Ward, White, Wolcott, Wang, Kuhn, Taylor, and John, "Development of a Hybrid Artificial Pancreas with Dense Polyurethane Membrane", ASAIO Journal, J.B. Lippincott, Vol. 39, No. 3, July-September 1993; Ward, White, Wang, and Wolcott, A Hybrid Artificial Pancreas with a Dense Polyurethane Membrane: Materials & Design, Proceedings of the 40th Anniversary Meeting of the American Society for Artificial Internal Organs, April 14-16,1994, San Francisco, California; Farrar, Litwak, Lawson, Ward, White, Robinson, Rodvien, and Hill, "In-Vivo Evaluation of a New Thromboresistant Polyurethane for Artificial Heart Blood Pumps", J. of Thoracic Surgery, 95:191-200, 1987; Jones, Soranno, Collier, Anderson, Ebert, Stokes, and Ward, Effects of Polyurethanes with SMEs on Fibroblast Adhesion and Proliferation and Monocyte and Macrophage Adhesion, The 28th Annual Meeting of the Society for Biomaterials, April 24-27, 2002, Tampa, Florida; and Ward, R.S. and White, K.A., Barrier Films that Breathe, CHEMTECH, November, 1991, 21(11), 670, all of which references are hereby expressly incorporated by reference.

Another embodiment of this invention is an article comprising a polymer body, wherein the polymer body comprises a plurality of polymer molecules located internally within the body, at least some of which internal polymer molecules have endgroups that comprise a surface of the body. In this embodiment, the surface endgroups include at least one surface-modifying endgroup moiety, provided that at least some of said covalently bonded surface-modifying endgroup moieties are other than alkylene ether-terminated poly(alkylene oxides). In accordance with this embodiment, the surface of the polymer body has enhanced antimicrobial properties, reduced aerodynamic or hydrodynamic drag, enhanced resistance to encrustation by marine organisms, and/or enhanced ability to release marine organisms when moving through water (e.g., ship's coatings), stealth properties, enhanced resistance to attachment of ice and/or enhanced ability to release ice when moving through air or water (e.g., ship or aircraft coatings), enhanced resistance to oxidation, corrosion, damage by sunlight, water, or other environmental degradation of the underlying substrate (e.g., exterior or interior paints, treatments, and protective coatings), reduced or enhanced coefficient of friction, enhanced surface lubricity, enhanced surface adhesion or tack, enhanced ease of donning, enhanced wear properties, enhanced abrasive properties, enhanced or reduced static dissipation, enhanced or reduced energy absorption and/or energy conversion (e.g., in photovoltaic applications), or enhanced or reduced responsiveness to temperature, pH, electricity, or other stimuli.

In one preferred aspect of this and other embodiments of the invention, the polymer includes a plurality of

endgroups each comprising a chain capable of self assembling, and also contains one or more head groups that ultimately reside in the outermost monolayer of the polymer's surface are that are optionally used in a coupling reaction to bind other moieties. In this and other embodiments, branched, star, dendritic, columnar, tubular, and/or other multi-armed polymer structures are optional features of the polymer to be modified.

In another embodiment of this invention, the self-assembling chains and/or the head groups of the endgroups include reactive sites for crosslinking the self-assembling chains to each other or to the base polymer, to minimize the ability of the modified-surface to restructure upon a change of environment, or when overcoated by an adsorbent. The latter is exemplified by, but not limited to, the use of an oleyl spacer chain between the polymer and the head group. This chain will self assemble in the surface in air and can subsequently be crosslinked by ultraviolet radiation, heat, or other means capable of inducing and/or catalyzing the reaction of double bonds. Once crosslinked, it is constrained from reorganizing, e.g., when immersed in an aqueous environment. Crosslinking, which may optionally include one or more additional reactants, initiators, inhibitors, or catalysts, immobilizes the self-assembled chains by joining them together with covalent chemical bonds or ionic bonds.

Before or after crosslinking the self-assembling spacer chains, the attached reactive head groups may be coupled to other optionally biologically-active moieties. A preferred approach for producing well-defined structures of this type is to use a different chemical reaction to crosslink the self-assembling spacer chains than the reaction used to couple active moieties to the head groups. A free radical or ionic reaction could, for instance, crosslink the spacer, preceding, following, or contemporaneously with a condensation reaction that couples an active moiety to the head group.

If the performance of the final surface in the intended application does not require a high level of coverage by the head groups, a mixture of head groups can be utilized in which some or all of the head groups take part in crosslinking reactions after self assembly of the spacer chains. For example, active hydrogen head groups could be reacted with appropriate polyfunctional crosslinkers. In another non-limiting example, acryloxy or methyacryloxy head groups may be linked together via free radical reactions, e.g., induced by heat or radiation (from UV or visible light, electron beam, gamma sources, etc.) in the presence of optional co-reactants. In still another examples, condensation reactions may be employed to crosslink the surface layer, for example by including silanes that give off a condensation byproducts such as water, acid, or alcohol during or prior to the formation of crosslinks. Such reactions may be externally catalyzed or self-catalyzed. For instance, self catalysis may occur when the condensation by-product is acetic acid. In certain cases, including free radial crosslinking of endgroups, inert environments may be needed to facilitate the crosslinking reaction. For example, shielding the surface reactions from oxygen via an inert gas blanket may be required during free radical reactions, whereas exposure to water may be required to initiate certain condensation crosslinking reactions involving silanes with multiple acyloxy groups used as reactive head groups. In addition to these examples, other suitable crosslinking reactions and reaction conditions can be chosen from the technical literature. These include a wide variety of well-known reactions commonly used for crosslinking polymer chains within the *bulk* of a formed article.

Crosslinking reactions may also be applied to the bulk polymer to be modified by the SAM-like SMEs. Crosslinking may be performed before, during, or after self assembly of the surface, to provide enhanced physical-mechanical properties, resistance to swelling, or any of the bulk property improvements associated with crosslinking that are well known to those skilled in the art. When the bulk polymer is t be crosslinked, it may be desirable to utilize spacer chains in the SME that do not crosslink, or which crosslink by a different mechanism, In this way, the bulk may be crosslinked before or after the surface spacer chains, without affecting the alignment or self-assembled structure of the spacer chains in the surface.

Yet another embodiment of this invention provides an article or device in which the nano surface architecture or micro surface architecture is a function of a variation in the chemical composition and molecular weight of surface-modifying endgroups to enhance or reduce cell adhesion to biomedical implants or to tissue engineering scaffolds.

The present invention has been illustrated by reference to certain specific embodiments thereof. However, those skilled in the art will readily appreciate that other, different embodiments can be practiced using the principles of the invention. All said embodiments constitute a part of the invention patented to the extent that they are reflected in the appended claims.

## Claims

**1.** A medical device or prosthesis or packaging assembly comprising a polymer body, wherein the polymer body comprises a plurality of polymer molecules located internally within said body, at least some of which internal polymer molecules have endgroups that comprise a surface of the body, wherein the surface endgroups include at least one self-assembling monolayer moiety, wherein the polymer comprising the self-assembling molecular moieties in the polymer body is a first polymer making up the entirety of a major portion of the body and having a weight average molecular weight in the range 5000-5,000,000 daltons, or is a second polymer, having a weight average molecular weight in the range 1000-500,000 daltons, which comprises an additive to the first polymer making up the entirety

or a major portion of the body.

2. The medical device or prosthesis or packaging assembly of claim 1, wherein said first polymer has a weight average molecular weight in the range 50,000-5,000,000 daltons.

3. The device or prosthesis of claim 1, configured as an implantable medical device or prosthesis or as a non-implantable disposable or extracorporeal medical device or prosthesis or as an *in vitro* or *in vivo* diagnostic device, wherein said device or prostheses has a tissue, fluid, and/or blood-contacting surface.

4. The device or prosthesis of claim 1, wherein said polymer body comprises a dense or microporous membrane component in an implantable medical device or prosthesis or in a non-implantable disposable or extracorporeal medical device or prosthesis or as an *in vitro* or *in vivo* diagnostic device, and wherein, when said polymer body comprises a membrane component in a diagnostic device, said component contains immuno-reactants.

5. The device or prosthesis of claim 1, wherein said device or prosthesis comprises a blood gas sensor, a compositional sensor, a substrate for combinatorial chemistry, a customizable active biochip, a semiconductor-based device for identifying and determining the function of genes, genetic mutations, and proteins, a drug discovery device, an immunochemical detection device, a glucose sensor, a pH sensor, a blood pressure sensor, a vascular catheter, a cardiac assist device, a prosthetic heart valve, an artificial heart, a vascular stent, a prosthetic spinal disc, a prosthetic spinal nucleus, a spine fixation device, a prosthetic joint, a cartilage repair device, a prosthetic tendon, a prosthetic ligament, a drug delivery device from which drug molecules are released over time, a drug delivery coating in which drugs are fixed permanently to polymer endgroups, a catheter balloon, a glove, a wound dressing, a blood collection device, a blood storage container, a blood processing device, a plasma filter, a plasma filtration catheter, a device for bone or tissue fixation, a urinary stent, a urinary catheter, a contact lens, an intraocular lens, an ophthalmic drug delivery device, a male condom, a female condom, devices and collection equipment for treating human infertility, a pacemaker lead, an implantable defibrillator lead, a neural stimulation lead, a scaffold for cell growth or tissue engineering, a prosthetic or cosmetic breast implant, a prosthetic or cosmetic pectoral implant, a prosthetic or cosmetic gluteus implant, a penile implant, an incontinence device, a laparoscope, a vessel or organ occlusion device, a bone plug, a hybrid artificial organ containing transplanted tissue, an *in vitro* or *in vivo* cell culture device, a blood filter, blood tubing, roller pump tubing, a cardiotomy reservoir, an oxygenator membrane, a dialysis membrane, an artificial lung, an artificial liver, or a column packing adsorbent or chelation agent for purifying or separating blood, plasma, or other fluids.

6. A drug delivery device in accordance with claim 1, wherein the drug is complexed to surface-modifying endgroups and is released through diffusion or wherein the drug is associated with, complexed to, or covalently bound to surface-modifying endgroups that degrade and release the drug over time.

7. A packaging assembly in accordance with claim 1 comprising a polymer body, wherein the polymer body comprises a plurality of polymer molecules located internally within said body, at least some of which internal polymer molecules have endgroups that comprise a surface of the body, wherein the surface endgroups include at least one self-assembling monolayer moiety,
wherein the polymer comprising the self-assembling monolayer moieties in the polymer body is a first polymer making up the entirety of a major portion of the body and having a weight average molecular weight in the range 5000-5,000,000 daltons, or is a second polymer, having a weight average molecular weight in the range 1000-500,000 daltons, which comprises an additive to the first polymer making up the entirety or a major portion of the body, or wherein said packaging assembly comprises a plastic bottle and eyedropper assembly containing a sterile solution, wherein said self-assembling monolayer moieties bind an antimicrobial agent and wherein said bound antimicrobial agents maintain the sterility of said solution.

8. The device or prosthesis of claim 1, configured as an implantable medical device or prosthesis or as a non-implantable disposable or extracorporeal medical device or prosthesis or as an *in vitro* or *in vivo* diagnostic device, wherein said device or prosthesis optionally has antimicrobial activity afforded by self-assembling antimicrobial agents covalently bonded to the polymer chain as an endgroup.

9. A method of immobilizing biologically-active entities, including proteins, peptides, and polysaccharides, at a surface of a polymer body, which polymer body surface comprises a surface of an interface, which method comprises the sequential steps of
contacting the polymer body surface with a medium that delivers self-assembling monolayer moieties containing

chemically-reactive groups, capable of binding biologically-active entities to the surface, to the polymer body surface by interaction of chemical groups, chains, or oligomers, said self-assembling monolayer moieties being covalently or ionically bonded to a polymer in the body and comprising one or more chemical groups, chains, or oligomers that spontaneously assemble in the outermost monolayer of the surface of the polymer body or one or more chemical groups, chains, or oligomers that spontaneously assemble within that portion of the polymer body that is at least one monolayer away form the outermost monolayer of the polymer body surface, and
binding said biologically-active entities to said reactive groups,
wherein the polymer comprising the self-assembling monolayer moieties in the polymer body is a first polymer making up the entirety of a major portion of the body and having a weight average molecular weight in the range 5000-5,000,000 daltons, or is a second polymer, having a weight average molecular weight in the range 1000-500,000 daltons, which comprises an additive to the first polymer making up the entirety or a major portion of the body, or
wherein said self-assembling monolayer moieties containing binding groups comprise methoxy ether-terminated polyethyleneoxide oligomers having one or more amino, hydroxyl, carboxaldehyde, or carboxyl groups along the polyethyleneoxide chain.

**10.** The method of immobilizing biologically-active entities according to claim 9, wherein the polymer comprising the self-assembling monolayer moieties in the polymer body is a first polymer making up the entirety of a major portion of the body and having a weight average molecular weight in the range 5000-5,000,000 daltons, or is a second polymer, having a weight average molecular weight in the range 1000-500,000 daltons, which comprises an additive to the first polymer making up the entirety or a major portion of the body.

**11.** The method of immobilizing biologically-active entities of claim 9, wherein said first polymer has a weight average molecular weight in the range 50,000-5,000,000 daltons.

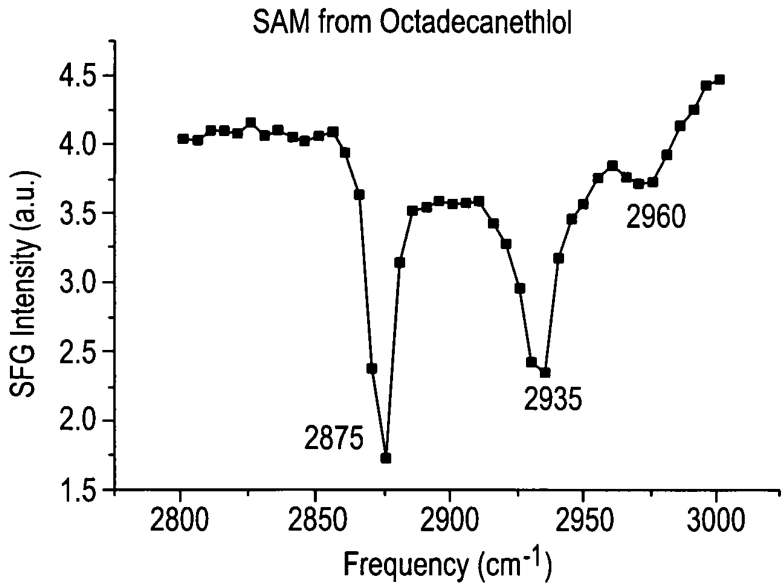


2875 - CH3 Symmetric
2935 - Fermi Resonance
2960 - CH3 Asymmetric

FIG.1A

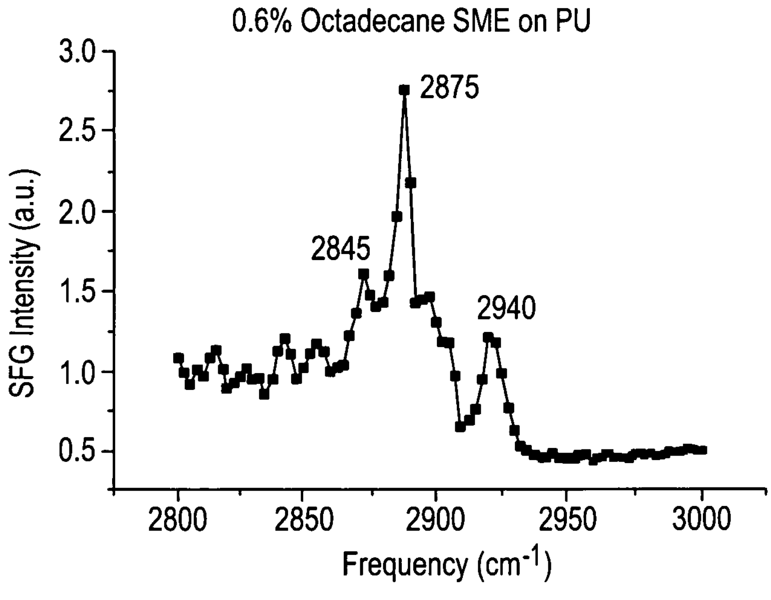


2845 - CH2 Symmetric (PU)
2875 - CH3 Symmetric (SME)
2940 - Fermi Resonance

FIG.1B

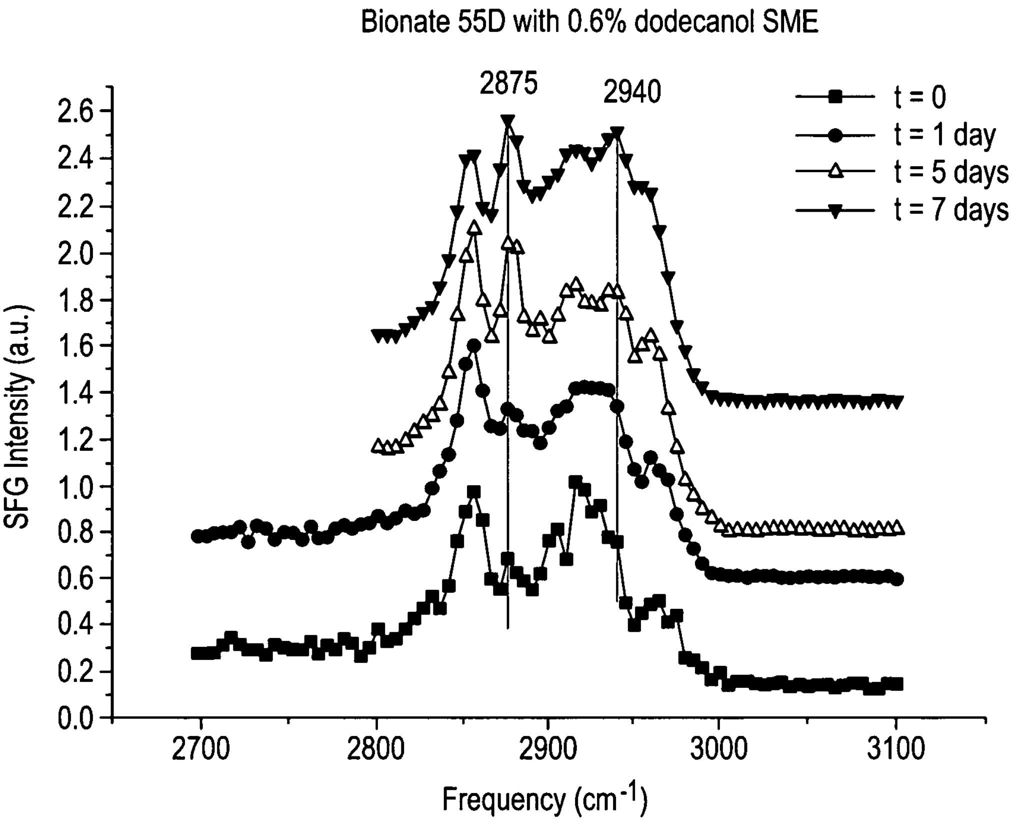
Bionate 55D with 0.6% dodecanol SME
2875
2940
t = 0
t = 1 day
t = 5 days
t = 7 days
SFG Intensity (a.u.)
2.6
2.4
2.2
2.0
1.8
1.6
1.4
1.2
1.0
0.8
0.6
0.4
0.2
0.0
2700
2800
2900
3000
3100
Frequency ($cm^{-1}$)

FIG.2

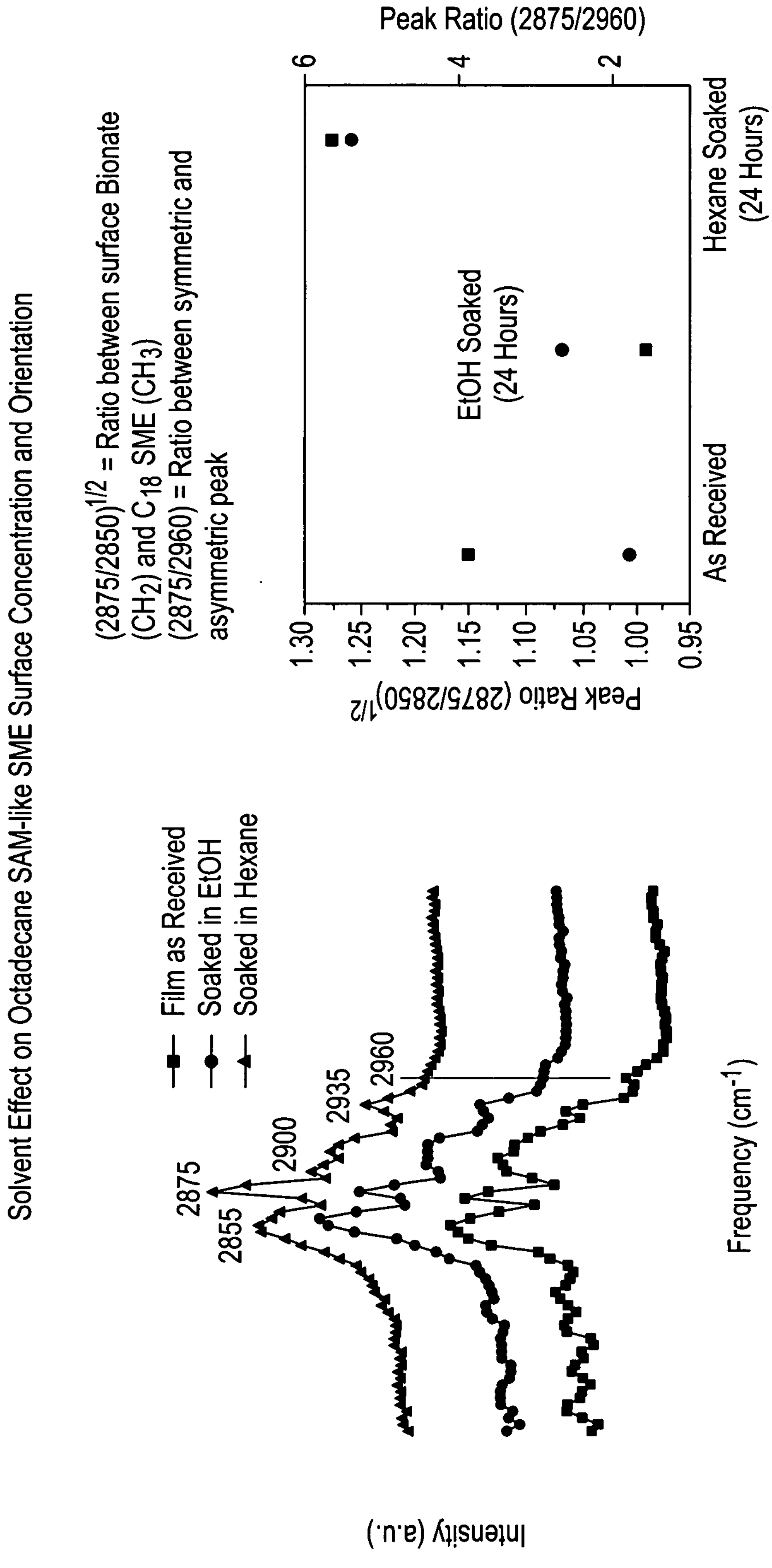

Solvent Effect on Octadecane SAM-like SME Surface Concentration and Orientation
Film as Received
Soaked in EtOH
Soaked in Hexane
2855
2875
2900
2935
2960
Intensity (a.u.)
Frequency ($cm^{-1}$)
$(2875/2850)^{1/2}$ = Ratio between surface Bionate ($CH_2$) and $C_{18}$ SME ($CH_3$)
(2875/2960) = Ratio between symmetric and asymmetric peak
Peak Ratio (2875/2960)
6
4
2
1.30
1.25
1.20
1.15
1.10
1.05
1.00
0.95
Peak Ratio $(2875/2850)^{1/2}$
As Received
EtOH Soaked (24 Hours)
Hexane Soaked (24 Hours)


FIG.3

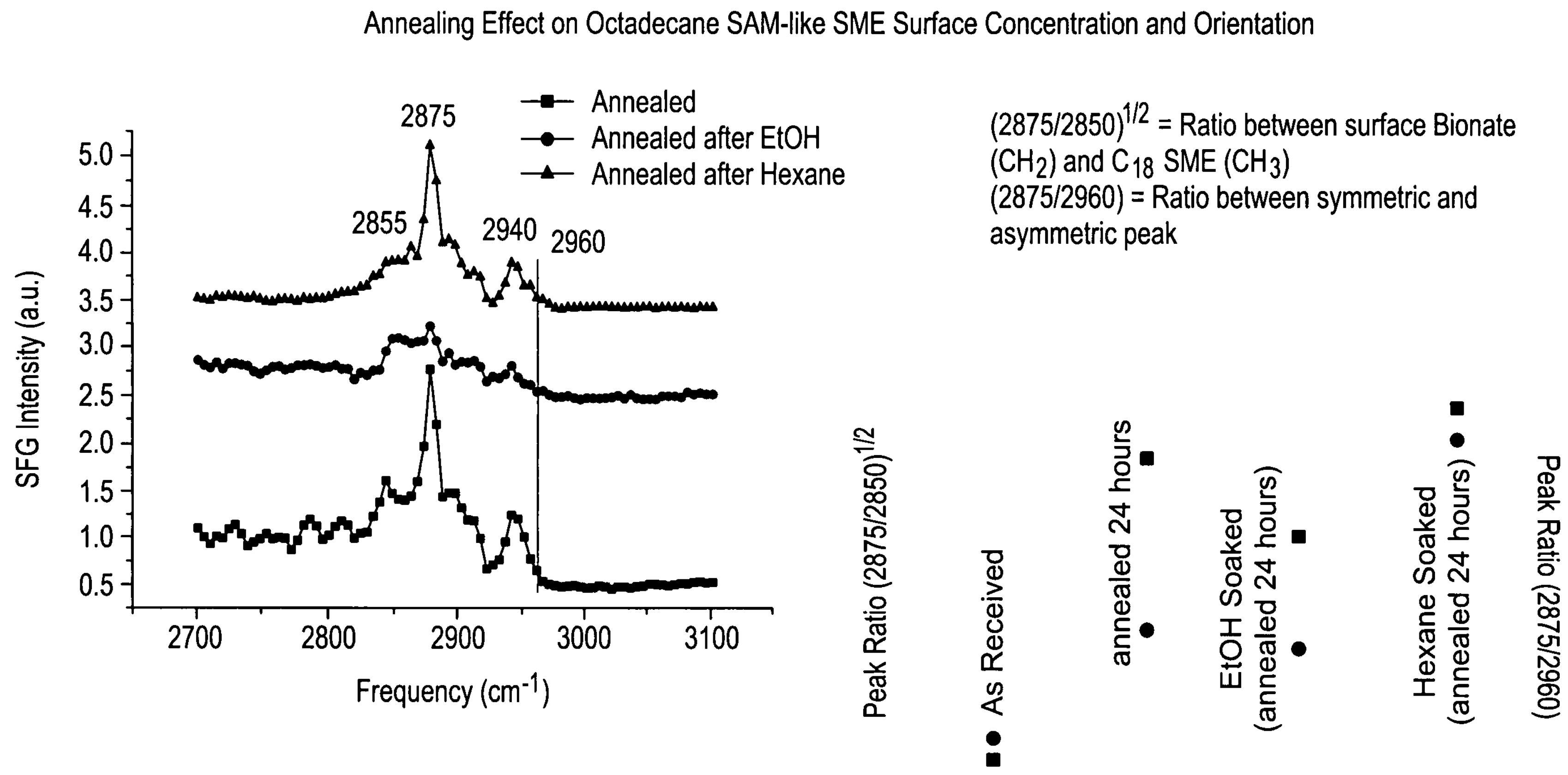
Annealing Effect on Octadecane SAM-like SME Surface Concentration and Orientation
Annealed
Annealed after EtOH
Annealed after Hexane
2875
2855
2940
2960
SFG Intensity (a.u.)
5.0
4.5
4.0
3.5
3.0
2.5
2.0
1.5
1.0
0.5
2700
2800
2900
3000
3100
Frequency (cm-1)
(2875/2850)1/2 = Ratio between surface Bionate (CH2) and C18 SME (CH3)
(2875/2960) = Ratio between symmetric and asymmetric peak
Peak Ratio (2875/2850)1/2
As Received
annealed 24 hours
EtOH Soaked (annealed 24 hours)
Hexane Soaked (annealed 24 hours)
Peak Ratio (2875/2960)

FIG.4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20050282997 A1
- US 11211734 B
- US 5589563 A
- WO 2004044012A1 A
- WO 2004044012 A1
- US 6492445 B2
- US 20050282977 A1


### Non-patent literature cited in the description


- **Ebert ; Stokes ; McVenes ; Ward ; Anderson.** Biostable Polyurethane Silicone Copolymers for Pacemaker Lead Insulation. *The 28th Annual Meeting of the Society for Biomaterials,* 24 April 2002
- **Ebert ; Stokes ; McVenes ; Ward ; Anderson.** Polyurethane Lead Insulation Improvements using Surface Modifying Endgroups. *The 28th Annual Meeting of the Society for Biomaterials,* 24 April 2002
- **Litwak ; Ward ; Robinson ; Yilgor ; Spatz.** Development of a Small Diameter, Compliant, Vascular Prosthesis. *Proceedings of the UCLA Symposium on Molecular and Cell Biology, Workshop on Tissue Engineering,* February 1988
- **Ward ; White ; Wolcott ; Wang ; Kuhn ; Taylor ; John.** Development of a Hybrid Artificial Pancreas with Dense Polyurethane Membrane. *ASAIO Journal, J.B. Lippincott,* July 1993, vol. 39 (3
- **Ward ; White ; Wang ; Wolcott.** A Hybrid Artificial Pancreas with a Dense Polyurethane Membrane: Materials & Design. *Proceedings of the 40th Anniversary Meeting of the American Society for Artificial Internal Organs,* 14 April 1994
- **Farrar ; Litwak ; Lawson ; Ward ; White ; Robinson ; Rodvien ; Hill.** In-Vivo Evaluation of a New Thromboresistant Polyurethane for Artificial Heart Blood Pumps. *J. of Thoracic Surgery,* 1987, vol. 95, 191-200
- **Jones ; Soranno ; Collier ; Anderson ; Ebert ; Stokes ; Ward.** Effects of Polyurethanes with SMEs on Fibroblast Adhesion and Proliferation and Monocyte and Macrophage Adhesion. *The 28th Annual Meeting of the Society for Biomaterials,* 24 April 2002
- **Ward, R.S. ; White, K.A.** Barrier Films that Breathe. *CHEMTECH,* November 1991, vol. 21 (11), 670